# EUROPEAN PATENT APPLICATION

(11) **EP 2 799 436 A1**
(43) Date of publication of application: **05.11.2014**
(21) Application number: 12861932.7
(22) Date of filing: 27.12.2012
(51) Int. Cl.: C07D 401/12, A61K 31/4192, A61K 31/454, A61P 3/04, A61P 3/06, A61P 3/10, A61P 11/00, A61P 11/02, A61P 25/08, A61P 25/14, A61P 25/18, A61P 25/20, A61P 25/24, A61P 25/26, A61P 25/28, A61P 37/08, A61P 43/00, C07D 401/14, C07D 403/12, C07D 403/14

(54) **PHENYLTRIAZOLE DERIVATIVE**

(30) Priority: 27.12.2011 JP 2011285731
(71) Applicant: Taisho Pharmaceutical Co., Ltd., Tokyo 170-8633 (JP)
(72) Inventor: NAKAMURA, Toshio, Tokyo 170-8633 (JP); MASUDA, Seiji, Tokyo 170-8633 (JP)
(74) Representative: Hoffmann Eitle
(86) International application number: PCT/JP2012/083882
(87) International publication number: WO 2013/100054

(57) **Abstract**

Provided are novel compounds that are useful in the prevention or treatment of such diseases as dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasomnia, sleep related movement disorder, insomnia, depression, or allergic rhinitis, or pharmaceutically acceptable salts of such compounds. Specifically, there are provided phenyltriazole derivatives represented by the following formula (I) or pharmaceutically acceptable salts thereof: 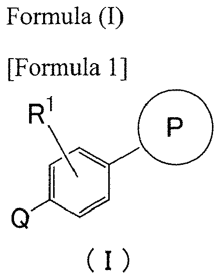 wherein ring P refers to a group represented by the following formula (II) or (III): 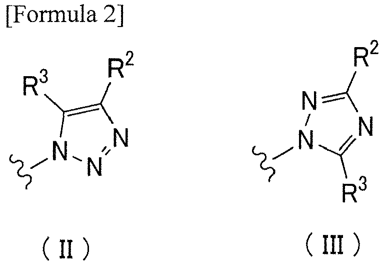 Q refers to a group represented by the following formula (A) or (B):

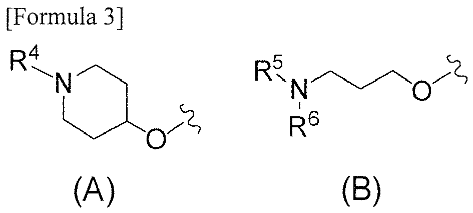

## Description

### TECHNICAL FIELD

The present invention relates to novel phenyltriazole derivatives and pharmaceutical uses thereof, in particular, preventive or therapeutic agents for diseases associated with histamine H3 receptors.

### BACKGROUND ART

Histamine is normally stored within intracellular granules in mast cells, lung, liver and gastric mucosa, etc. and released from the cell in response to external stimuli such as antigen-binding to an antibody on the cell surface. For example, when mast cells are stimulated by an antigen entering from outside, histamine is released from the mast cells and stimulates histamine H1 (H1) receptors located on blood vessels or smooth muscles, thereby triggering an allergic reaction. Likewise, histamine released from ECL cells (enterochromaffin-like cells) on the gastric mucosa stimulates histamine H2 (H2) receptors on the parietal cells to promote gastric acid secretion. Based on these facts, H1 and H2 receptor antagonists have been developed as therapeutic agents for allergic diseases and gastric ulcer, respectively, and currently find wide use as medicaments.

It has been found that histamine serves as a neurotransmitter and acts on histamine receptors (histamine H3 (H3) receptors) located on the central and peripheral nerves to exhibit various physiological functions. This receptor was cloned in 1999, and its gene sequence and amino acid sequence were determined. However, the amino acid sequence of H3 receptor has only 22% and 21.4% homology with those of H1 receptor and H2 receptor, respectively (see Non-Patent Document 1). H3 receptors which are present in the presynaptic membrane have been shown to serve as autoreceptors that control the synthesis and release of histamine (see Non-Patent Document 2). In addition to that, H3 receptors have been shown to control the release of other neurotransmitters including acetylcholine, serotonin, dopamine, and noradrenaline (see Non-Patent Document 3). It has also been suggested that H3 receptors are active in the absence of agonists and this activity is able to be inhibited by compounds acting as inverse agonists. These facts suggest that H3 receptor antagonists or inverse agonists enhance the release of H3 receptor-controlled neurotransmitters and may potentially serve as therapeutic agents for various diseases associated with abnormal release thereof.

As a matter of fact, results of experiments with animal models show the possibility that H3 receptor antagonists or inverse agonists can be used as therapeutic agents for dementia, Alzheimer's disease (see Non-Patent Documents 4 and 5), attention-deficit hyperactivity disorder (see Non-Patent Document 6), schizophrenia (see Non-Patent Document 7), epilepsy, central convulsion, etc.

It has been shown that H3 receptors are involved in eating behavior (see Non-Patent Document 8) and metabolic diseases including obesity, diabetes mellitus, hyperlipidemia, etc. are also assumed as diseases for which H3 receptor antagonists or inverse agonists are indicated.

It has been shown that histamine regulates the circadian rhythm in the brain and is responsible for maintaining the balance between waking and sleeping states (see Non-Patent Documents 9 and 10) and diseases associated with sleep disorders, including narcolepsy, sleep apnea syndrome, circadian rhythm disorder, and depression, are also assumed as diseases for which H3 receptor antagonists or inverse agonists are indicated.

It has been shown that H3 receptors are present in sympathetic nerves on the nasal mucosa, and reported that the combined use of H3 and H1 receptor antagonists improved nasal congestion significantly (see Non-Patent Document 11). This indicates the possibility that H3 receptor antagonists or inverse agonists are useful for treatment of such diseases as allergic rhinitis when they are used either alone or in combination with H1 receptor antagonists.

Outlines of H3 receptor antagonists or inverse agonists are found in several reviews (see Non-Patent Documents 12 to 15) to which reference may be made. In earlier years, there have been reported many imidazole compounds which were derived from histamine itself as a lead compound. However, those have yet to be developed as medicaments due to the concerns of the inhibition of the drug-metabolizing enzyme cytochrome P450 (CYP).

In recent years, non-imidazole H3 receptor antagonists or inverse agonists have been reported in many documents and patents (see Patent Documents 1 to 10).

Reports have also been made of histamine H3 receptor antagonists having 5-membered aromatic rings such as the pyrazole ring (see Patent Documents 11 to 15). However, there has been no report about compounds having the structures disclosed hereinafter.

### CITATION LIST

### PATENT DOCUMENTS

Patent Document 1: International Patent Publication WO2005097751
Patent Document 2: International Patent Publication WO2005097778
Patent Document 3: International Patent Publication WO2005118547
Patent Document 4: International Patent Publication WO2006014136
Patent Document 5: International Patent Publication WO2006045416
Patent Document 6: International Patent Publication WO2006046131
Patent Document 7: International Patent Publication WO2006059778
Patent Document 8: International Patent Publication WO2006061193
Patent Document 9: International Patent Publication WO2006107661
Patent Document 10: International Patent Publication WO2006103057
Patent Document 11: International Patent Publication WO2006103045
Patent Document 12: International Patent Publication WO2007094962
Patent Document 13: International Patent Publication WO2008072724
Patent Document 14: International Patent Publication WO2009063953
Patent Document 15: International Patent Publication WO2002012190

### NON-PATENT DOCUMENTS

Non-Patent Document 1: Lovenberg T.W. et al., Molecular pharmacology, 55, 1101-1107, 1999
Non-Patent Document 2: Arrang J-M. et al., Nature, 302, 832-837, 1983
Non-Patent Document 3: Brown R.E. et al., Progress in Neurobiology, 63, 637-672, 2001
Non-Patent Document 4: Huang Y-W. et al., Behavioural Brain Research, 151, 287-293,2004
Non-Patent Document 5: Komater V.A. et al., Behavioural Brain Research, 159, 295-300, 2005
Non-Patent Document 6: Passani M.B. et al., Neuroscience and Biobehavioral Reviews, 24, 107-113,2000
Non-Patent Document 7: Fox G.B. et al., J. Pharmacol. Exp. Ther., 313,176-190, 2005
Non-Patent Document 8: Hancock A.A. et al., Curr. Opin. Investig. Drug, 4, 1190-1197
Non-Patent Document 9: Huang Z-L. et al., Prog. Natr. Acad. Sci., 103, 4687-4692, 2006
Non-Patent Document 10: Babier A.J. et al., Br. J. Pharmacol., 143, 649-661, 2004
Non-Patent Document 11: McLeod R.L. et al., Am. J. Rhinol., 13, 391-399, 1999
Non-Patent Document 12: Schwartz J.C. et al., Trends in Pharmacol. Sci., 7, 24-28, 1986
Non-Patent Document 13: Passani M.B. et al., Trends in Pharmacol. Sci., 25, 618-625,2004
Non-Patent Document 14: Leurs R. et al., Nature Drug Discovery, 4, 107-122, 2005
Non-Patent Document 15: Leurs R. et al., Drug Discovery Today, 10, 1613-1627, 2005

### SUMMARY OF INVENTION

### TECHNICAL PROBLEM

The present invention has as an object providing novel compounds or their pharmaceutically acceptable salts, which have a potent action for inhibiting the binding of histamine to the histamine H3 receptor and which are useful in the prevention or treatment of disorders due to the histamine H3 receptor, for example, such diseases as dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasominia, sleep related movement disorder, insomnia, depression, or allergic rhinitis.

### SOLUTION TO PROBLEM

To attain the above-stated object, the present inventors conducted intensive studies and found as a result that a compound having a phenyltriazole skeleton (hereinafter referred to as a "phenyltriazole derivative") exhibited potent inhibitory activity against the binding of histamine to the histamine H3 receptor. This finding has led to the completion of the present invention.

Hereinafter, the present invention will be described in detail. Embodiments of the present invention (compound embodiments will be called "the inventive compounds") are as shown below.

Briefly, the present invention relates to:
(1) A compound represented by formula (I): 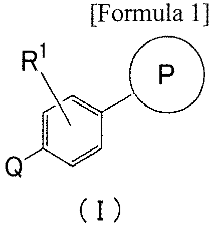 [wherein
   ring P refers to a group represented by the following formula (II) or (III): 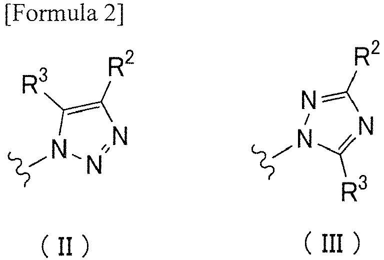 Q refers to a group represented by the following formula (A) or (B): 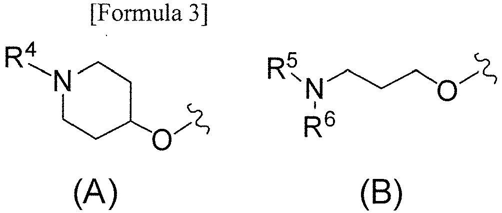 R¹ is a hydrogen atom, a halogen atom, or C₁-C₆ alkyl;
   R² is a hydrogen atom, hydroxy, cyano, carboxy, C₁-C₆ alkyl, C₂-C₇ alkanoyl, C₁-C₆ alkylsulfonyl (the C₁-C₆ alkyl, C₂-C₇ alkanoyl or C₁-C₆ alkylsulfonyl may be substituted by one to three groups selected from the group consisting of a halogen atom, hydroxy and C₁-C₆ alkoxy), C₁-C₆ alkoxy, C₃-C₇ cycloalkyl, C₂-C₇ alkoxycarbonyl (the C₁-C₆ alkoxy, C₃-C₇ cycloalkyl or C₂-C₇ alkoxycarbonyl may be substituted by one to three groups selected from the group consisting of a halogen atom, hydroxy, C₁-C₆ alkyl and C₁-C₆ alkoxy), -(CH₂)ₙ-C(=O)NR^{1A}R^{1B}, or -S(=O)₂NR^{2A}R^{2B};
   R^{1A} and R^{1B}, which may be the same or different, are each a hydrogen atom, C₁-C₆ alkyl or C₃-C₇ cycloalkyl, or
   R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl);
   n is 0 or 1;
   R^{2A} and R^{2B}, which may be the same or different, are each a hydrogen atom, C₁-C₆ alkyl or C₃-C₇ cycloalkyl, or
   R^{2A} and R^{2B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl);
   R³ is a hydrogen atom or C₁-C₆ alkyl;
   R⁴ is C₁-C₆ alkyl (the C₁-C₆ alkyl may be substituted by one or two C₃-C₇ cycloalkyls) or C₃-C₇ cycloalkyl (the C₃-C₇ cycloalkyl may be substituted by one or two C₁-C₆ alkyls); and
   R⁵ and R⁶, which may be the same or different, are each C₁-C₆ alkyl or C₃-C₇ cycloalkyl, or R⁵ and R⁶ may be bonded together with the adjacent nitrogen atom to form a 3- to 7-membered saturated heterocyclic ring (the saturated heterocyclic ring being optionally substituted by one or two C₁-C₆ alkyls)] (provided that N,N-dimethyl-3-[4-(1H-1,2,4-triazol-1-yl)phenoxy]propan-1-amine is excluded) or a pharmaceutically acceptable salt thereof.
(2) A compound according to (1), wherein the ring P is represented by formula (II): 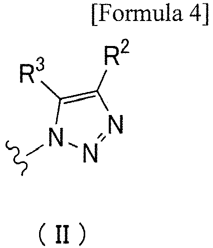 (wherein R² and R³ are as defined in (1)) or a pharmaceutically acceptable salt thereof.
(3) A compound according to (1) or (2), wherein Q is represented by formula (A): 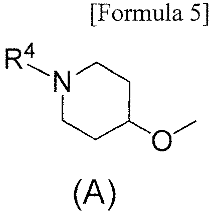 (wherein R⁴ is as defined in (1)) or a pharmaceutically acceptable salt thereof.
(4) A compound according to any one of (1) to (3), wherein R¹ is a hydrogen atom, or a pharmaceutically acceptable salt thereof.
(5) A compound according to any one of (1) to (4), wherein
   R² is C₁-C₆ alkyl (the C₁-C₆ alkyl may be substituted by one to three groups selected from the group consisting of hydroxy and C₁-C₆ alkoxy), C₁-C₆ alkoxy, C₃-C₇ cycloalkyl (the C₁-C₆ alkoxy or C₃-C₇ cycloalkyl may be substituted by one to three groups selected from the group consisting of hydroxy, C₁-C₆ alkyl and C₁-C₆ alkoxy) or -C(=O)NR^{1A}R^{1B}; and R^{1A} and R^{1B}, which may be the same or different, are each a hydrogen atom, C₁-C₆ alkyl or C₃-C₇ cycloalkyl, or
   R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl), or a pharmaceutically acceptable salt thereof.
(6) A compound according to any one of (1) to (5), wherein R³ is a hydrogen atom, or a pharmaceutically acceptable salt thereof.
(7) A compound according to any one of (1) to (6), wherein R⁴ is C₃-C₇ cycloalkyl, or a pharmaceutically acceptable salt thereof.
(8) A pharmaceutical agent comprising as the active ingredient a compound according to any one of (1) to (7) or a pharmaceutically acceptable salt thereof.
(9) A pharmaceutical agent according to (8) which is a histamine H3 receptor antagonist or inverse agonist.
(10) A pharmaceutical agent according to (8) or (9) which is a preventive or therapeutic agent for dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasomnia, sleep related movement disorder, insomnia, depression, or allergic rhinitis.

### ADVANTAGEOUS EFFECTS OF INVENTION

The compounds of the present invention have an outstanding histamine H3 receptor antagonistic action.

### DESCRIPTION OF EMBODIMENTS

The terms and expressions used herein are as defined below.

The "halogen atom" as used herein refers to a fluorine atom, a chlorine atom, a bromine atom, or an iodine atom.

The "C₁-C₆ alkyl" as used herein refers to a linear or branched alkyl group having 1 to 6 carbon atoms and may be exemplified by such groups as methyl, ethyl, n-propyl, isopropyl, n-butyl, isobutyl, sec-butyl, tert-butyl, n-pentyl, isopentyl, neopentyl, or n-hexyl.

The "C₃-C₇ cycloalkyl" as used herein may be exemplified by such groups as cyclopropyl, cyclobutyl, cyclopentyl, cyclohexyl, or cycloheptyl.

The "C₁-C₆ alkoxy" as used herein refers to a linear or branched alkoxy group having 1 to 6 carbon atoms and may be exemplified by such groups as methoxy, ethoxy, n-propoxy, isopropoxy, n-butoxy, isobutoxy, sec-butoxy, tert-butoxy, n-pentyloxy, isopentyloxy, neopentyloxy, or n-hexyloxy.

The "C₂-C₇ alkanoyl" as used herein refers to a carbonyl group attached to an alkyl group having 1 to 6 carbon atoms and may be exemplified by such groups as acetyl, propionyl, butyryl, isobutyryl, pivaloyl, pentanoyl, 3-methylbutyryl, 4,4-dimethylpentanoyl, or heptanoyl.

The "C₂-C₇ alkoxycarbonyl" as used herein refers to a carbonyl group attached to a linear or branched alkoxy group having 1 to 6 carbon atoms and may be exemplified by such groups as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl, isopentyloxycarbonyl, neopentyloxycarbonyl, or n-hexyloxycarbonyl.

The "C₁-C₆ alkylsulfonyl" as used herein refers to a linear or branched alkylsulfonyl group having 1 to 6 carbon atoms and may be exemplified by such groups as methylsulfonyl, n-propylsulfonyl, isobutylsulfonyl, or n-hexylsulfonyl.

The "3- to 7-membered saturated heterocyclic ring" in the expression "bonded together with the adjacent nitrogen atom to form a 3- to 7-membered saturated heterocyclic ring" refers to a saturated monocyclic ring or spiro ring that is composed of 3 to 7 ring forming atoms and which contains said adjacent nitrogen atom, with optional additional inclusion of a single hetero atom selected from among O, N, and S; examples may include such groups as 1-aziridinyl, 1-azetidinyl, 1-pyrrolidinyl, piperidino, 1-azepanyl, or morpholino.

Preferred embodiments of the inventive compounds are shown below.

One preferred embodiment of the compound of formula (I) according to the present invention is where the ring P is represented by formula (II): 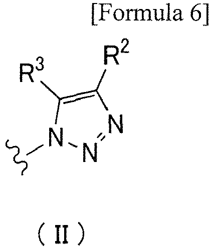 (wherein
R² is a hydrogen atom, hydroxy, cyano, carboxy, C₁-C₆ alkyl, C₂-C₇ alkanoyl, C₁-C₆ alkylsulfonyl (the C₁-C₆ alkyl, C₂-C₇ alkanoyl or C₁-C₆ alkylsulfonyl may be substituted by one to three groups selected from the group consisting of a halogen atom, hydroxy and C₁-C₆ alkoxy), C₁-C₆ alkoxy, C₃-C₇ cycloalkyl, C₂-C₇ alkoxycarbonyl (the C₁-C₆ alkoxy, C₃-C₇ cycloalkyl or C₂-C₇ alkoxycarbonyl may be substituted by one to three groups selected from the group consisting of a halogen atom, hydroxy, C₁-C₆ alkyl and C₁-C₆ alkoxy), -(CH₂)ₙ-C(=O)NR^{1A}R^{1B}, or -S(=O)₂NR^{2A}R^{2B};
R^{1A} and R^{1B}, which may be the same or different, are each a hydrogen atom, C₁-C₆ alkyl or C₃-C₇ cycloalkyl, or
R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C_{I}-C₆ alkyl);
n is 0 or 1;
R^{1A} and R^{2B}, which may be the same or different, are each a hydrogen atom, C₁-C₆ alkyl or C₃-C₇ cycloalkyl, or
R^{2A} and R^{2B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl); and
R³ is a hydrogen atom or C₁-C₆ alkyl.)

In formula (II), R² is preferably C₁-C₆ alkyl (the C₁-C₆ alkyl may be substituted by one to three groups selected from the group consisting of hydroxy and C₁-C₆ alkoxy), C₁-C₆ alkoxy, C₃-C₇ cycloalkyl (the C₁-C₆ alkoxy or C₃-C₇ cycloalkyl may be substituted by one to three groups selected from the group consisting of hydroxy, C₁-C₆ alkyl and C₁-C₆ alkoxy) or -C(=O)NR^{1A}R^{1B}, wherein R^{1A} and R^{1B}, which may be the same or different, are each a hydrogen atom, C₁-C₆ alkyl or C₃-C₇ cycloalkyl; alternatively, R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl).

In formula (II), R³ is preferably a hydrogen atom.

Another preferred embodiment of the compound of formula (I) according to the present invention is where Q is represented by formula (A): 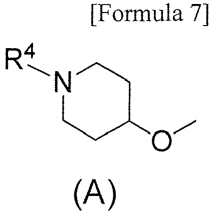 (wherein R⁴ is C₁-C₆ alkyl (the C₁-C₆ alkyl may be substituted by one or two C₃-C₇ cycloalkyls) or C₃-C₇ cycloalkyl (the C₃-C₇ cycloalkyl may be substituted by one or two C₁-C₆ alkyls)).
In formula (A), R⁴ is preferably C₃-C₇ cycloalkyl, more preferably cyclobutyl.

Still another preferred embodiment of the compound of formula (I) according to the present invention is where R¹ is a hydrogen atom.

Profiles the compounds of the present invention preferably have include high drug efficacy, superior in vivo kinetics (good oral absorption and no accumulation in particular tissues), superior properties exhibited as pharmaceuticals, low toxicity, etc. Preferred compounds of the present invention are less likely to be recognized as a substrate for P-glycoprotein which is an efflux transporter that controls intracerebral migration of drugs and hence, those compounds are expected to have superior intracerebral migration.

The "pharmaceutically acceptable salt" as used herein encompasses, for example, salts with inorganic acids such as sulfuric acid, hydrochloric acid, hydrobromic acid, phosphoric acid, and nitric acid; salts with organic acids such as acetic acid, oxalic acid, succinic acid, lactic acid, tartaric acid, fumaric acid, maleic acid, citric acid, benzenesulfonic acid, methanesulfonic acid, p-toluenesulfonic acid, benzoic acid, camphorsulfonic acid, ethanesulfonic acid, glucoheptonic acid, gluconic acid, glutamic acid, glycolic acid, malic acid, malonic acid, mandelic acid, galactaric acid, and naphthalene-2-sulfonic acid; salts with one or more metal ions such as lithium ion, sodium ion, potassium ion, calcium ion, magnesium ion, zinc ion, and aluminum ion; and salts with ammonia or amines such as arginine, lysine, piperazine, choline, diethylamine, 4-phenylcyclohexylamine, 2-aminoethanol, and benzathine.

The compounds of the present invention may also occur in the form of various solvates. They may sometimes be in a hydrate form from the viewpoint of applicability as pharmaceuticals.

The compounds of the present invention encompass all possible forms including enantiomers, diastereomers, equilibrium compounds, mixtures thereof in any proportions, racemates, and so on. Individual isomers can be obtained by known methods, for example, use of an optically active starting material or intermediate, optically selective or diastereoselective reaction in the production of an intermediate or the final product, or chromatographic separation in the production of an intermediate or the final product.

The compounds of the present invention also encompass those in which one or more hydrogen atoms, carbon atoms, nitrogen atoms, oxygen atoms, sulfur atoms, halogen atoms, etc. are replaced by their radioisotopes or stable isotopes. These labeled compounds are useful in, for example, studies of metabolism and pharmacokinetics, or biological analyses in which they are used as receptor ligands.

The compounds of the present invention or pharmaceutically acceptable salts thereof may be combined with one or more pharmaceutically acceptable carriers, excipients or diluents to formulate pharmaceutical preparations. Such carriers, excipients and diluents may include, for example, water, lactose, dextrose, fructose, sucrose, sorbitol, mannitol, polyethylene glycol, propylene glycol, starch, gum, gelatin, alginate, calcium silicate, calcium phosphate, cellulose, water syrup, methylcellulose, polyvinylpyrrolidone, alkyl parahydroxybenzosorbate, talc, magnesium stearate, stearic acid, glycerin, and various kinds of oil such as sesame oil, olive oil, and soybean oil.

Moreover, the above-mentioned carriers, excipients or diluents may optionally be blended with commonly used additives such as extenders, binders, disintegrants, pH modifiers, solubilizers, etc. and then processed by usual pharmaceutical formulating procedures to prepare oral or parenteral pharmaceuticals such as tablets, pills, capsules, granules, dusts, liquids/solutions, emulsions, suspensions, ointments, injections, and skin plasters. The compounds of the present invention may be given to adult patients at doses of 0.001 to 500 mg per administration, once or several times a day, by the oral or parenteral route. This dosage may be increased or decreased as appropriate for the type of disease to be treated, the age, body weight and symptom of the patient, and so on.

Pharmaceuticals containing the compounds of the present invention or pharmaceutically acceptable salts thereof as the active ingredient are useful as histamine H3 receptor antagonists or inverse agonists.

What is more, pharmaceuticals containing the compounds of the present invention or pharmaceutically acceptable salts thereof as the active ingredient are useful as preventive or therapeutic agents for dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasomnia, sleep related movement disorder, insomnia, depression, or allergic rhinitis.

The compounds of the present invention can be produced by known techniques in organic chemistry. Methods according to the reaction schemes shown below are exemplary processes for producing the compounds of the present invention and are by no means intended to limit the same. In Reaction Schemes 1 to 4 set out below, R¹, R², R³, R⁴, R⁵, R⁶, R^{1A}, R^{1B} and n are as defined above. In addition, X, Y¹, Y² and Y³, which may be the same or different, each represent a leaving group such as a halogen atom (e.g., a chlorine atom, a bromine atom, or an iodine atom) or an organic sulfonyloxy group (e.g., a methanesulfonyloxy group, a benzenesulfonyloxy group, a p-toluenesulfonyloxy group, or a trifluoromethanesulfonyloxy group), or a hydroxyl group.

Described below is the process for producing a compound of the present invention that is depicted by Reaction Scheme 1. This is a process for producing a compound (IA) of the present invention from a compound (1).

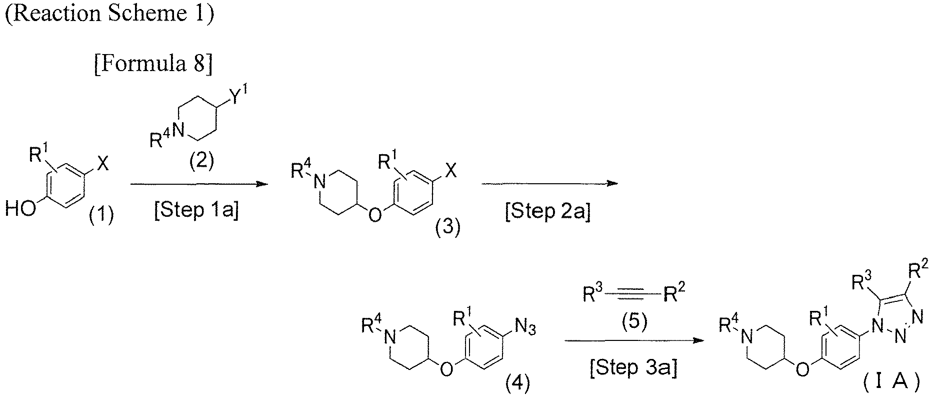

### (Step 1a)

Step 1a is for achieving condensation between the compound (1) and a compound (2) through coupling reaction to form a compound (3). The compounds (1) and (2) are either known or can be readily synthesized from known compounds.

In the case where Y¹ is a leaving group such as a halogen atom or an organic sulfonyloxy group, the coupling reaction may be carried out by a common method involving alkylation of the hydroxy group of phenol either in a solvent or without a solvent in the presence or absence of a base. If necessary, an additive may be added, as exemplified by potassium iodide or sodium bromide. Examples of the base that may be used in the reaction under consideration include organic bases such as pyridine, triethylamine, and diisopropylethylamine; alkali metal alkoxides such as potassium tert-butoxide; and inorganic bases such as potassium carbonate, cesium carbonate, sodium hydrogencarbonate, sodium hydroxide, potassium hydroxide, and sodium hydride. Examples of the solvent that may be used in the reaction under consideration include alcohols such as methanol, ethanol, and 2-propanol; ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. The reaction temperature for the reaction under consideration generally ranges from 0°C to 200°C, preferably from 15°C to 150°C, and the reaction time generally ranges from 1 to 48 hours, preferably from 1 to 16 hours.

In the case where Y¹ is a hydroxyl group, the coupling reaction under consideration may be exemplified by the Mitsunobu reaction; an example of the method for carrying out this reaction is one that is performed in a solvent in the presence of a reagent comprising an organophosphorus compound such as triphenylphosphine or tributylphosphine combined with an azo compound such as diethyl azodicarboxylate, diisopropyl azodicarboxylate, or di-tert-butyl azodicarboxylate, or alternatively, in the presence of a phosphorus ylide reagent such as cyanomethylene tributyl phosphorane. Examples of the solvent that may be used in the reaction under consideration include ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; or mixed solvents thereof. The reaction temperature for the reaction under consideration generally ranges from 0°C to 150°C, preferably from 15°C to 100°C, and the reaction time generally ranges from 1 to 48 hours, preferably from 1 to 16 hours.

### (Step 2a)

Step 2a is for converting the compound (3) to an azide compound (4). This conversion reaction can be carried out by a common method for the reaction in a solvent in the presence of sodium azide, a catalyst and its ligand; for instance, it can be carried out according to the method described in Liang et al., Synlett, 2005, Vol. 14, pp. 2209-2213 or a modification thereof. The reaction under consideration is preferably performed in the presence of a base. Examples of the catalyst that may be used in the reaction under consideration include a copper catalyst, and more specifically they include copper(0), copper(I) iodide, copper(I) chloride, copper(I) oxide, copper(I) bromide, and copper(II) sulfate. The ligand that may be used in the reaction under consideration is selected from ligands that are commonly used in reaction using a copper catalyst and examples include N,N'-dimethylethylenediamine, N,N'-dimethylcyclohexane-1,2-diamine, 2-aminopyridine, 1,10-phenanthroline, 3,4,7,8-tetramethyl-1,10-phenanthroline, 2-hydroxybenzaldehyde oxime, ethylene glycol, triphenylphosphine, and tri-tert-butylphosphine. Examples of the base that may be used in the reaction under consideration include potassium carbonate, potassium phosphate, potassium hydroxide, potassium tert-butoxide, sodium tert-butoxide, cesium carbonate, sodium ascorbate, sodium carbonate, sodium hydroxide, sodium hydrogencarbonate, sodium acetate, sodium methoxide, and tetrabutylammonium hydroxide. Examples of the solvent that may be used in the reaction under consideration include alcohols such as methanol, ethanol, and 2-propanol; ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. The reaction temperature for the reaction under consideration generally ranges from 0°C to 200°C, preferably from 40°C to 150°C, and the reaction time generally ranges from 30 minutes to 24 hours, preferably from 30 minutes to 6 hours.

### (Step 3a)

Step 3a is for producing the inventive compound (IA) through cycloaddition reaction between the compound (4) and a compound (5). The compound (5) is either known or can be readily synthesized from a known compound. The reaction under consideration can be carried out by a common method for the reaction either in a solvent or without a solvent; for instance, it can be carried out according to the method described in Synthesis, 2011, pp. 223-228 or a modification thereof. The reaction under consideration can be carried out in the presence of a catalyst, a ligand and a base. Examples of the catalyst that may be used in the reaction under consideration include a copper catalyst, and more specifically they include copper(0), copper(I) iodide, copper(I) chloride, copper(I) oxide, copper(I) bromide, and copper(II) sulfate. The ligand that may be used in the reaction under consideration is selected from ligands that are commonly used in reaction using a copper catalyst and examples include N,N'-dimethylethylenediamine, N,N'-dimethylcyclohexane-1,2-diamine, 2-aminopyridine, 1,10-phenanthroline, 3,4,7,8-tetramethyl-1,10-phenanthroline, 2-hydroxybenzaldehyde oxime, ethylene glycol, triphenylphosphine, and tri-tert-butylphosphine. Examples of the base that may be used in the reaction under consideration include potassium carbonate, potassium phosphate, potassium hydroxide, potassium tert-butoxide, sodium tert-butoxide, cesium carbonate, sodium ascorbate, sodium carbonate, sodium hydroxide, sodium hydrogencarbonate, sodium acetate, sodium methoxide, and tetrabutylammonium hydroxide. Examples of the solvent that may be used in the reaction under consideration include alcohols such as methanol, ethanol, and 2-propanol; ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. The reaction temperature for the reaction under consideration generally ranges from 0°C to 200°C, preferably from 40°C to 150°C, and the reaction time generally ranges from 30 minutes to 48 hours, preferably from 30 minutes to 6 hours.

A compound (IA-2) can be produced from a compound (IA-1) of the present invention according to the method depicted by Reaction Scheme 2.

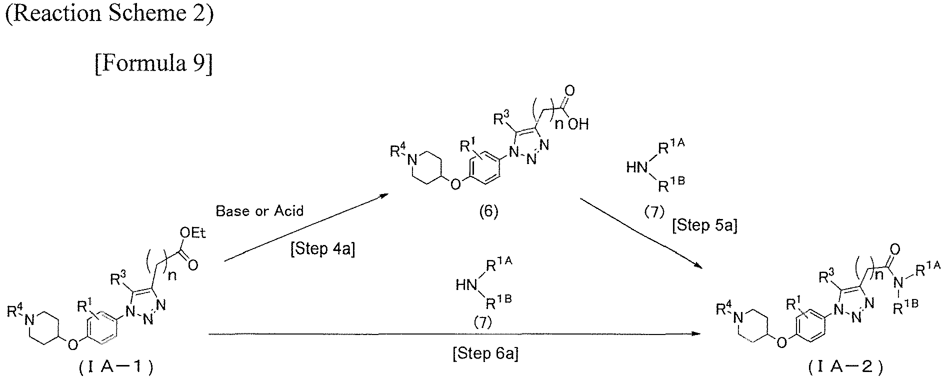

### (Step 4a)

Step 4a is such that the ethoxycarbonyl group of the inventive compound (IA-1) is converted to a carboxylic acid through hydrolysis to form an inventive compound (6) in which R² is a carboxy group. The hydrolysis reaction can be carried out through a common reaction of ester hydrolysis; for instance, it can be carried out according to the methods described in T. W. Greene and P. G. M. Wuts, Protective Groups in Organic Synthesis, fourth edition, John Wiley and Sons or modifications thereof, as exemplified by a method of performing the reaction either in a solvent or without a solvent in the presence of a strong acid, and a method of performing the reaction in a solvent in the presence of a base. The reaction temperature for the reaction under consideration generally ranges from 0°C to 120°C, preferably from 15°C to 80°C, and the reaction time generally ranges from 1 to 48 hours, preferably from 1 to 12 hours.

### (Step 5a)

Step 5a is for achieving condensation between the carboxylic acid compound (6) and an amine compound (7) through coupling reaction to form the inventive compound (IA-2). The compound (7) is either known or can be readily synthesized from a known compound. The coupling reaction can be carried out by common methods of amidating carboxylic acids, which include a method in which a carboxylic acid is converted to a carboxylic acid halide such as carboxylic acid chloride or carboxylic acid bromide and then reacted with amine, a method in which a mixed acid anhydride as obtained from a carboxylic acid and chlorocarbonic acid ester is reacted with amine, a method in which a carboxylic acid is converted to an active ester such as 1-benzotriazolyl ester or succinimidyl ester and then reacted with amine, and a method in which a carboxylic acid is reacted with amine in the presence of a dehydration condensation agent. All of these reactions may be performed in a solvent in the presence or absence of a base. Examples of the dehydration condensation agent that may be used in the reaction under consideration include 3-(3-dimethylaminopropyl)-1-ethylcarbodiimide hydrochloride, dicyclohexylcarbodiimide, diphenylphosphorylazide, and carbonyldiimidazole, with an activator such as 1-hydroxybenzotriazole or hydroxysuccinimide being optionally used. Examples of the base that may be used in the reaction under consideration include pyridine, triethylamine, diisopropylethylamine, potassium carbonate, sodium carbonate, and sodium hydrogencarbonate. Examples of the solvent that may be used in the reaction under consideration include ethers such as tetrahydrofuran and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. Among them, preferred is toluene, tetrahydrofuran or N,N-dimethylformamide. The reaction temperature for the reaction under consideration generally ranges from 0°C to 120°C, preferably from 15°C to 40°C, and the reaction time generally ranges from 1 to 48 hours, preferably from 1 to 12 hours.

### (Step 6a)

Step 6a is for obtaining the compound (IA-2) through ester-amide exchange reaction between the inventive compound (IA-1) and the amine compound (7). All of this reaction may be performed either without a solvent or in a solvent in the presence or absence of a base. If necessary, an additive may be added, as exemplified by aluminum chloride or sodium methoxide. Examples of the solvent that may be used in the reaction under consideration include alcohols such as methanol, ethanol, and 2-propanol; ethers such as tetrahydrofuran and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. Among them, preferred is toluene, tetrahydrofuran or N,N-dimethylformamide. The reaction temperature for the reaction under consideration generally ranges from 0°C to 120°C, preferably from 40°C to 120°C, and the reaction time generally ranges from 1 to 48 hours, preferably from 1 to 12 hours.

Described below is the process for producing a compound of the present invention that is depicted by Reaction Scheme 3. This is a process for producing a compound (IB) of the present invention from the compound (1).

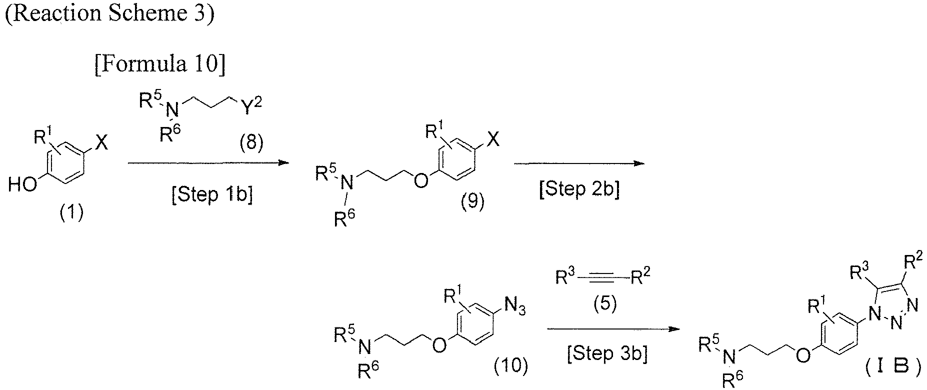

### (Step 1b)

Step 1 b is for obtaining a compound (9) through coupling reaction between the compound (1) and a compound (8). The compound (8) is either known or can be readily synthesized from a known compound. The coupling reaction can be carried out by the same method as in Step 1a.

### (Step 2b)

Step 2b is for obtaining a compound (10) from the compound (9). This reaction can be carried out by the same method as in Step 2a.

### (Step 3b)

Step 3b is for obtaining the compound (IB) through cycloaddition reaction between the compound (10) and the compound (5). This reaction can be carried out by the same method as in Step 3a.

Described below is the process for producing a compound of the present invention that is depicted by Reaction Scheme 4. This is a process for producing a compound (IB-2) of the present invention from a compound (IB-1).

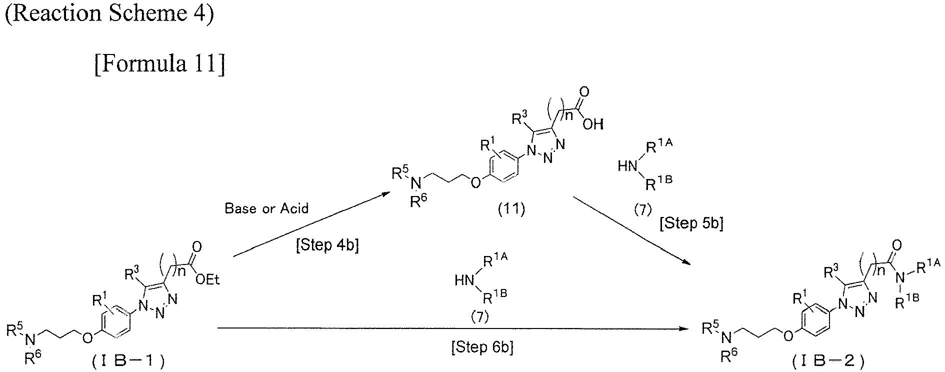

### (Step 4b)

Step 4b is such that the ethoxycarbonyl group of the compound (IB-1) is converted to a carboxylic acid through hydrolysis to form an inventive compound (11) in which R² is a carboxy group. This reaction can be carried out by the same method as in Step 4a.

### (Step 5b)

Step 5b is for achieving condensation between the carboxylic acid compound (11) and the amine compound (7) through coupling reaction to form the inventive compound (IB-2). This reaction can be carried out by the same method as in Step 5a.

### (Step 6b)

Step 6b is for obtaining the compound (IB-2) through ester-amide exchange reaction between the inventive compound (IB-1) and the amine compound (7). This reaction can be carried out by the same method as in Step 6a.

Described below is the process for producing a compound of the present invention that is depicted by Reaction Scheme 5. This is a process for producing a compound (IC) of the present invention from a compound (12).

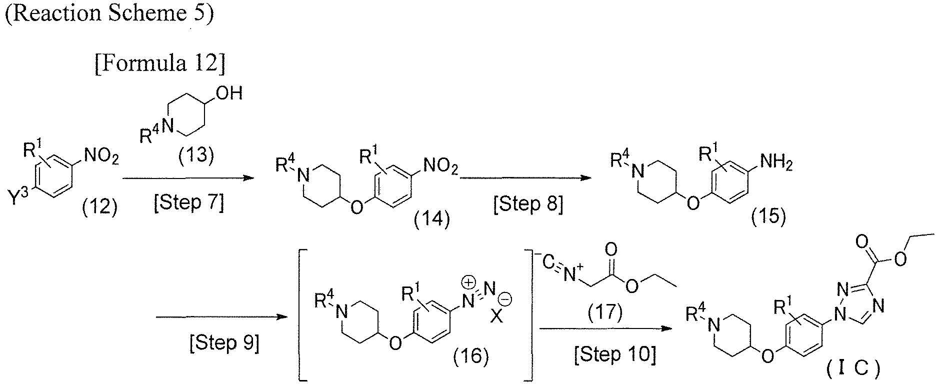

Step 7 is for obtaining a compound (14) from the compound (12) and a compound (13). The compounds (12) and (13) are either known or can be readily synthesized from known compounds. The reaction under consideration can be carried out through a common reaction of aromatic nucleophilic substitution; for instance, it can be carried out according to the method described in the Journal of Medicinal Chemistry, 2008, Vol. 51, p. 6889 or a modification thereof. The conditions that may be set in the reaction under consideration include a method for the reaction either in a solvent or without a solvent in the presence or absence of a base. Examples of the base that may be used in the reaction under consideration include organic bases such as pyridine, triethylamine, and diisopropylethylamine; alkali metal alkoxides such as potassium tert-butoxide; and inorganic bases such as potassium carbonate, cesium carbonate, sodium hydrogencarbonate, sodium hydroxide, potassium hydroxide, and sodium hydride. Examples of the solvent that may be used in the reaction under consideration include ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. The reaction temperature for the reaction under consideration generally ranges from 0°C to 200°C, preferably from 15°C to 150°C, and the reaction time generally ranges from 1 to 48 hours, preferably from 1 to 16 hours.

Step 8 is for obtaining a compound (15) from the compound (14). This reaction can be carried out through reaction of the compound (14) in a solvent under conditions for reduction reaction; for instance, it can be carried out according to the method described in the Journal of American Chemical Society, 1944, Vol. 66, p. 1442 or a modification thereof. Examples of the reduction reaction conditions that may be set in the reaction under consideration include a method for performing the reaction by adding a catalyst such as Raney Nickel or palladium carbon under a hydrogen atmosphere either at normal pressure or under pressure, a method for the reaction with a metal hydrogen complex compound such as lithium aluminum hydride or sodium borohydride, a method for the reaction with iron(0), zinc(II) chloride or tin(II) chloride in the presence of an acid such as acetic acid or ammonium chloride, or combinations of these conditions. Examples of the solvent that may be used in the reaction under consideration include alcohols such as methanol and ethanol; ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; or mixed solvents thereof. The reaction temperature for the reaction under consideration generally ranges from 0°C to 200°C, preferably from 15°C to 150°C, and the reaction time generally ranges from 1 to 48 hours, preferably from 1 to 16 hours.

Step 9 is for obtaining a compound (16) from the compound (15). This reaction can be carried out through a common reaction of diazotization; for instance, it can be carried out according to the method described in the Journal of Organic Chemistry, 2005, Vol. 70, p. 1050 or a modification thereof. The conditions that may be set in the reaction under consideration include a method for reaction between a nitrite salt such as sodium nitrite or a nitrite ester such as isoamyl nitrite and the compound (15) either in a solvent or without a solvent in the presence of an acid. Examples of the acid that may be used in the reaction under consideration include inorganic acids such as hydrochloric acid, hydrobromic acid and sulfuric acid, and Lewis acids such as boron trifluoride, trimethyl aluminum and aluminum chloride. Examples of the solvent that may be used in the reaction under consideration include water; alcohols such as methanol and ethanol; ethers such as tetrahydrofuran, 1,2-dimethoxyethane, and 1,4-dioxane; hydrocarbons such as toluene and benzene; halogenated hydrocarbons such as chloroform and dichloromethane; or mixed solvents thereof. The reaction temperature for the reaction under consideration generally ranges from -10°C to 200°C, preferably from -10°C to 50°C, and the reaction time generally ranges from 15 minutes to 48 hours, preferably from 15 minutes to 5 hours.

Step 10 is for obtaining the compound (IC) from the compound (16). This reaction can be carried out through cyclization reaction between the diazo compound (16) and an isonitrile compound (17). The conditions that may be set in the reaction under consideration include the reaction performed either in a solvent or without a solvent in the presence or absence of a base. Examples of the base that may be used in the reaction under consideration include organic bases such as pyridine, triethylamine, and diisopropylethylamine; alkali metal alkoxides such as potassium tert-butoxide; and inorganic bases such as sodium acetate, potassium acetate, potassium carbonate, cesium carbonate, sodium hydrogencarbonate, sodium hydroxide, and potassium hydroxide. Examples of the solvent that may be used in the reaction under consideration include ethers such as tetrahydrofuran, 1,2-dimethoxyethane, and 1,4-dioxane; hydrocarbons such as toluene and benzene; halogenated hydrocarbons such as chloroform and dichloromethane; amides such as N,N-dimethylformamide, N,N-dimethylacetamide, and N-methyl-2-pyrrolidone; ketones such as acetone and 2-butanone; dimethyl sulfoxide; acetonitrile; water; or mixed solvents thereof. The reaction temperature for the reaction under consideration generally ranges from 0°C to 200°C, preferably from 15°C to 150°C, and the reaction time generally ranges from 1 to 48 hours, preferably from 1 to 16 hours.

Described below is the process for producing a compound of the present invention that is depicted by Reaction Scheme 6. This is a process for producing a compound (IC-1) of the present invention from the compound (IC).

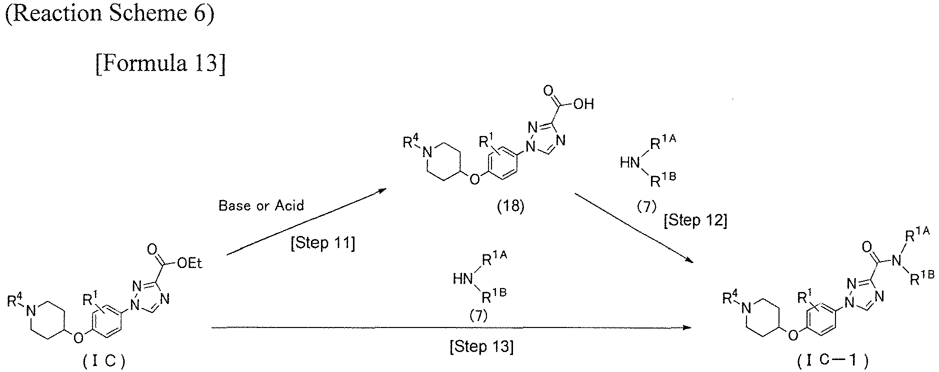

### (Step 11)

Step 11 is such that the ethoxycarbonyl group of the compound (IC) is converted to a carboxylic acid through hydrolysis to form an inventive compound (18). This reaction can be carried out by the same method as in Step 4a.

### (Step 12)

Step 5b is for achieving condensation between the carboxylic acid compound (18) and the amine compound (7) through coupling reaction to form the inventive compound (IC-1). This reaction can be carried out by the same method as in Step 5a.

### (Step 13)

Step 13 is for obtaining the compound (IC-1) through ester-amide exchange reaction between the inventive compound (IC) and the amine compound (7). This reaction can be carried out by the same method as in Step 6a.

### EXAMPLES

On the following pages, the present invention is described specifically by means of working examples and tests, which are not intended to limit the scope of the invention.

The instrument data listed in the working examples were obtained by measurement with the following instruments:
MS spectrum: micromass Platform LC or micromass GCT
NMR spectrum: [¹H-NMR] 600 MHz: JNM-ECA600 (JEOL Ltd., Japan)

### (Example 1)

### Preparation of ethyl 1-{4-[(1-cyclobutylpiperidin-4-yl)oxy]phenyl}-1H-1,2,3-triazole-4-carboxylate (Compound No. 1)

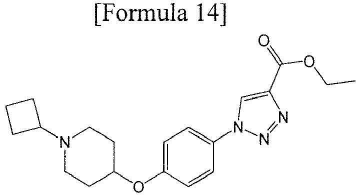

A mixture of 1-cyclobutyl-4-(4-iodophenoxy)piperidine (1.0 g, which can be synthesized according to the method described in WO2008072703), sodium azide (0.32 g), N,N'-dimethylethylenediamine (0.049 g), copper iodide (0.053 g), sodium ascorbate (0.027 g), ethanol (9.0 mL) and water (1.0 mL) was stirred at 70°C for 2 hours. The reaction mixture was left to cool to room temperature, water was added, and the mixture was extracted with chloroform. The organic layer was concentrated under reduced pressure to give an azide compound based mixture (0.83 g).
MS (ESI pos.) m/z : 273 [M+H]⁺

A suspension of the residue (0.80 g), ethyl propiolate (576 mg), copper iodide (55.9 mg) and sodium ascorbate (116 mg) in toluene (5.0 mL) was stirred in a sealed tube at 70°C. To the reaction mixture, saturated aqueous sodium hydrogencarbonate was added, and the mixture was extracted with ethyl acetate. The organic layer was dried over sodium sulfate and then concentrated under reduced pressure. The resulting residue was purified by silica gel column chromatography (NH form silica gel; eluent: n-hexane/ethyl acetate = 80/20 - 20/80) to give the titled compound as a colorless solid (0.271 g).
1H NMR (600 MHz, CHLOROFORM-d) δ ppm 1.44 (t, J=7.22 Hz, 3 H) 1.64 - 1.77 (m, 2 H) 1.80 - 1.95 (m, 4 H) 1.98 - 2.09 (m, 4 H) 2.12 - 2.26 (m, 2 H) 2.63 (br. s., 2 H) 2.75 (t, J=7.64 Hz, 1 H) 4.39 (br. s., 1 H) 4.47 (q, J=7.16 Hz, 2 H) 7.01 - 7.06 (m, 2 H) 7.60 - 7.65 (m, 2 H) 8.41 (s, 1 H); MS (ESI pos.) m/z : 371 [M+H]⁺

The same procedure as described in Example 1 was repeated to give the compounds listed below in Table 1 (Compound Nos. 2 to 7).

**[Table 1]**

| Compound No. | Structural Formula | Compound Name | Instrument Data |
|---|---|---|---|
| 2 | 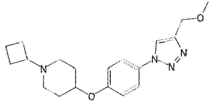 | 1-cyclobutyl-4-{4-[4-(methoxymethyl)-1H-1,2,3-triazol-1-yl]phenoxy}piperidine | 1H NMR (600 MHz, CHLOROFORM-d) *δ* ppm 1.64 - 1.76 (m, 2 H) 1.82 - 1.96 (m, 4 H) 1.99 - 2.10 (m, 4 H) 2.14-2.28 (m, 2 H) 2.64 (br. s., 2 H) 2.76 (d, J=7.84 Hz, 1 H) 3.47 (s, 3 H) 4.39 (br. s., 1 H) 4.66 (s, 2 H) 6.99 - 7.03 (m, 2 H) 7.58 - 7.62 (m, 2 H) 7.88 (s, 1 H); MS (ESI pos.) m/z : 343 [M+H]⁺ |
| 3 | 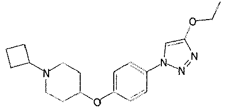 | 1-cyclobutyl-4-[4-(4-ethoxy-1H-1,2,3-triazol-1-yl)phenoxy] piperidine | 1H NMR (600 MHz, CHLOROFORM-d) *δ* ppm 1.45 (t, J=7.02 Hz, 3 H) 1.63 - 1.76 (m, 2 H) 1.81 - 1.96 (m, 4 H) 1.99 - 2.09 (m, 4 H) 2.14 - 2.25 (m, 2 H) 2.63 (br. s., 2 H) 2.72 - 2.79 (m, 1 H) 4.33 - 4.41 (m, 3 H) 6.98 - 7.02 (m, 2 H) 7.32 (s, 1 H) 7.55 - 7.59 (m, 2 H); MS (ESI pos.) m/z : 343 [M+H]⁺ |
| 4 | 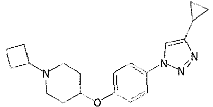 | 1-cyclobutyl-4-[4-(4-cyclopropyl-1H-1,2,3-triazol-1-yl)phenoxy] piperidine | 1H NMR (600 MHz, CHLOROFORM-d) *δ* ppm 0.88 - 0.92 (m, 2 H) 0.96 - 1.01 (m, 2 H) 1.63 - 1.75 (m, 2 H) 1.80 - 1.95 (m, 4 H) 1.97 - 2.08 (m, 5 H) 2.14 - 2.26 (m, 2 H) 2.62 (br. s., 2 H) 2.76 (br. s., 1H) 4.36 (br. s., 1 H) 6.96 - 7.00 (m, 2 H) 7.53 - 7.58 (m, 3 H); MS (ESI pos.) m/z : 339 [M+H]+ |
| 5 | 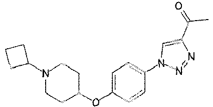 | 1-(1-{4-[(1-cyclobutylpiperidin-4-yl)oxy]phenyl}-1H-1,2,3-triazol-4-yl)ethanone | 1H NMR (600 MHz, CHLOROFORM-d) *δ* ppm 1.64 - 1.77 (m, 2 H) 1.82 - 1.96 (m, 4 H) 2.05 (d, J=7.02 Hz, 4 H) 2.19 (d, J=17.75 Hz, 2 H) 2.64 (br. s., 2 H) 2.75 (s, 4 H) 4.40 (br. s., 1H) 7.01 - 7.06 (m, 2 H) 7.60 - 7.65 (m, 2 H) 8.39 (s, 1 H); MS (ESI pos.) m/z : 341 [M+H]⁺ |
| 6 | 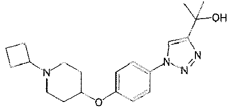 | 2-(1-{4-[(1-cyclobutylpiperidin-4-yl)oxy]phenyl}-1H-1,2,3-triazol-4-yl)propan-2-ol | 1H NMR (600 MHz, CHLOROFORM-d) *δ* ppm 1.64 - 1.75 (m, 8 H) 1.81 - 1.94 (m, 4 H) 1.99 - 2.09 (m, 4 H) 2.17 (br. s., 2 H) 2.41 (s, 1 H) 2.64 (br. s., 2 H) 2.75 (t, J=7.84 Hz, 1 H) 4.38 (br. s., 1 H) 6.99 - 7.03 (m, 2 H) 7.57 - 7.61 (m, 2 H) 7.79 (s, 1 H); MS (ESI pos.) m/z : 357 [M+H]+ |
| 7 | 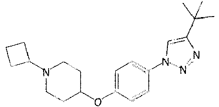 | 4-[4-(4-tert-butyl-1H-1,2,3-triazol-1-yl)phenoxy]-1-cyclobutylpiperidine | 1H NMR (600 MHz, CHLOROFORM-d) *δ* ppm 1.41 (s, 9 H) 1.65 - 1.76 (m, 2 H) 1.81 - 1.96 (m, 4 H) 2.00 - 2.09 (m, 4 H) 2.21 (br. s., 2 H) 2.64 (br. s., 2 H) 2.73 - 2.81 (m, 1 H) 4.38 (br. s., 1 H) 7.00 (d, J=8.67 Hz, 2 H) 7.57 - 7.61 (m, 3 H); MS (ESI pos.) m/z : 355 [M+H]+ |

### (Example 2)

### Preparation of (1-{4-[(1-cyclobutylpiperidin-4-yl)oxy]phenyl}-1H-1,2,3-triazol-4-yl)(pyrrolidin-1-yl)methanone (Compound No. 8)

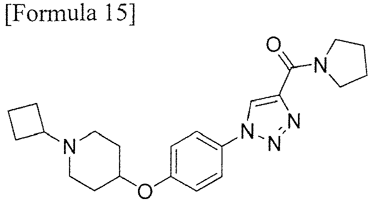

A mixture of the ethyl 1-{4-[(1-cyclobutylpiperidin-4-yl)oxy]phenyl}-1H-1,2,3-triazole-4-carboxylate (0.070 g) synthesized in Example 1 and pyrrolidine (2.0 mL) was stirred in a sealed tube at 90°C for 5 hours. The reaction mixture was left to cool to room temperature, saturated aqueous sodium hydrogencarbonate was added, and the mixture was extracted with ethyl acetate. The organic layer was dried over sodium sulfate and then concentrated under reduced pressure. The resulting residue was purified by silica gel column chromatography (NH form silica gel; eluent: n-hexane/ethyl acetate = 80/20 - 0/100) to give the titled compound as a colorless solid (0.022 g).
1H NMR (600 MHz, CHLOROFORM-d) δ ppm 1.64 - 1.77 (m, 2 H) 1.80 - 1.98 (m, 6 H) 1.98 - 2.10 (m, 6 H) 2.19 (br. s., 2 H) 2.64 (br. s., 2 H) 2.75 (d, J=7.84 Hz, 1 H) 3.70 (t, J=7.02 Hz, 2 H) 4.18 (t, J=6.81 Hz, 2 H) 4.39 (br. s., 1 H) 7.00 - 7.06 (m, 2 H) 7.59 - 7.66 (m, 2 H) 8.45 (s, 1 H); MS (ESI pos.) m/z : 396 [M+H]⁺
The same procedure as described in Example 2 was repeated to give the compounds listed below in Tables 2-1 and 2-2 (Compound Nos. 9 to 16).

**[Table 2-1]**

| Compound No. | Structural Formula | Compound Name | Instrument Data |
|---|---|---|---|
| 9 | 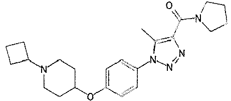 | (1-{4-[(1-cyclobutylpiperidin-4-yl)oxy]phenyl}-5-methyl-1H-1,2,3-triazol-4-yl)(pyrrolidin-1-yl)methanone | 1H NMR (600 MHz, CHLOROFORM-d) *δ* ppm 1.64-1.77 (m,2H)1.81-2.11 (m, 12 H) 2.18 (d, J=8.67 Hz, 2 H) 2.55 (s, 3 H) 2.64 (br. s., 2 H) 2.71 - 2.80 (m, 1H) 3.69 (t, J=7.02 Hz, 2 H) 4.11 (t, J=6.81 Hz, 2 H) 4.40 (br. s., 1 H) 7.02 - 7.06 (m, 2 H) 7.33 (d, J=8.67 Hz, 2 H); MS (ESI pos.) m/z : 410 [M+H]+ |
| 10 | 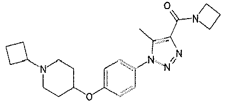 | azetidin-1-yl(1-{4-[(1-cyclobutylpiperidin-4-yl)oxy]phenyl}-5-methyl-1H-1,2,3-triazol-4-yl)methanone | 1H NMR (600 MHz, CHLOROFORM-d) *δ* ppm 1.64-1.77 (m, 2H) 1.81 - 1.94 (m, 4 H) 1.98 - 2.10 (m, 4 H) 2.13 - 2.24 (m, 2 H) 2.36 - 2.42 (m, 2 H) 2.57 (s, 3 H) 2.60 - 2.69 (m, 2 H) 2.71 - 2.78 (m, 1H) 4.23 (t, J=7.64 Hz, 2 H) 4.40 (br. s., 1 H) 4.77 (t, J=7.64 Hz, 2 H) 7.01 - 7.05 (m, 2 H) 7.32 (d, J=8.67 Hz, 2 H); MS (ESI pos.) m/z : 396 [M+H]⁺ |
| 11 | 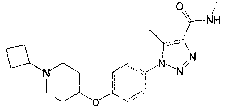 | 1-{4-[(1-cyclobutylpiperidin-4-yl)oxy]phenyl}-N,5-dimethyl-1H-1,2,3-triazole-4-carboxamide | 1H NMR (600 MHz, CHLOROFORM-d) *δ* ppm 1.64 - 1.76 (m, 2 H) 1.83 - 1.94 (m, 4 H) 2.00 - 2.09 (m, 4 H) 2.14 - 2.24 (m, 2 H) 2.59 (s, 3 H) 2.61 - 2.69 (m, 2 H) 2.72 - 2.79 (m, 1 H) 3.02 (d, J=4.95 Hz, 3 H) 4.36 - 4.44 (m, 1H) 7.04 (d, J=9.08 Hz, 2 H) 7.33 (d, J=9.08 Hz, 2 H); MS (ESI pos.) m/z : 370 [M+H]⁺ |
| 12 | 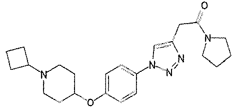 | 2-(1-{4-[(1-cyclobutylpiperidin-4-yl) oxy]phenyl}-1H-1,2,3-triazol-4-yl)-1-(pyrrolidin-1-yl)ethanone | 1H NMR (600 MHz, CHLOROFORM-d) *δ* ppm 1.62 - 1.92 (m, 8 H) 1.96 - 2.09 (m, 6 H) 2.18 (d, J=6.19 Hz, 2 H) 2.59 - 2.68 (m, 2 H) 2.75 (t, J=7.84 Hz, 1 H) 3.50 (t, J=6.81 Hz, 2 H) 3.64 (t, J=6.81 Hz, 2 H) 3.87 (s, 2 H) 4.37 (br. s., 1H) 6.97 - 7.02 (m, 2 H) 7.58 - 7.63 (m, 2 H) 8.08 (s, 1H); MS (ESI pos.) m/z : 410 [M+H]+ |
| 13 | 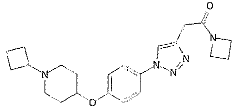 | 1-(azetidin-1-yl)-2-(1-{4-[(1-cyclobutylpiperidin-4-yl)oxy]phenyl}-1H-1,2,3-triazol-4-yl)ethanone | 1H NMR (600 MHz, CHLOROFORM-d) *δ* ppm 1.64 - 1.78 (m, 2 H) 1.80 - 1.95 (m, 4 H) 1.96 - 2.10 (m, 4 H) 2.12 - 2.24 (m, 2 H) 2.26 - 2.34 (m, 2 H) 2.57 - 2.69 (m, 2 H) 2.71 - 2.80 (m, 1H) 3.64 - 3.66 (m, 2 H) 4.06 (t, J=7.84 Hz, 2 H) 4.31 - 4.41 (m, 3 H) 6.98 - 7.02 (m, 2 H) 7.58 - 7.62 (m, 2 H) 8.03 (s, 1 H); MS (ESI pos.) m/z : 396 [M+H]+ |
| 14 | 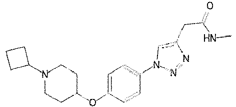 | 2-(1-{4-[(1-cyclobutylpiperidin-4-yl) oxy]phenyl}-1H-1,2,3-triazol-4-yl)-N-methylacetamide | 1H NMR (600 MHz, CHLOROFORM-d) *δ* ppm 1.65-1.76 (m,2H)1.81-1.94 (m, 4 H) 1.98 - 2.09 (m, 4 H) 2.17 (br. s., 2 H) 2.63 (br. s., 2 H) 2.71 - 2.78 (m, 1H) 2.84 (d, J=4.54 Hz, 3 H) 3.75 (s, 2 H) 4.35 - 4.42 (m, 1 H) 6.50 (br. s., 1 H) 6.99 - 7.04 (m, 2 H) 7.59 (d, J=9.08 Hz, 2 H) 7.86 (s, 1H); MS (ESI pos.) m/z : 370 [M+H]⁺ |

**[Table 2-2]**

| Compound No. | Structural Formula | Compound Name | Instrument Data |
|---|---|---|---|
| 15 | 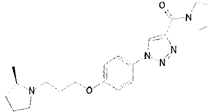 | [1-(4-{3-[(2R)-2-methylpyrrolidin-1 -yl]propoxy}phenyl)-1H-1,2,3-triazol-4-yl](pyrrolidin-1-yl)methanone | 1H NMR (600 MHz, CHLOROFORM-d) *δ* ppm 1.09 (d, J=6.19 Hz, 3 H) 1.42 (br. s., 1 H) 1.66 - 1.74 (m, 1 H) 1.75 - 1.83 (m, 1 H) 1.89 - 1.97 (m, 3 H) 1.99 - 2.07 (m, 4 H) 2.09 - 2.15 (m, 1 H) 2.17 - 2.25 (m, 1 H) 2.27 - 2.35 (m, 1 H) 2.95 - 3.03 (m, 1 H) 3.18 (t, J=7.64 Hz, 1 H) 3.70 (t, J=7.02 Hz, 2 H) 4.06 - 4.12 2 (m, 2 H) 4.18 (t, J=6.81 Hz, 2 H) 7.02 - 7.06 (m, 2 H) 7.61 - 7.66 (m, 2 H) 8.46 (s, 1 H); MS (ESI pos.) m/z : 384 [M+H]+ |
| 16 | 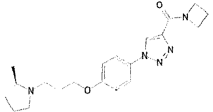 | azetidin-1-yl[1-(4-{3-[(2R)-2-methylpyrrolidin-1-yl]propoxy}phenyl)-1H-1,2,3-triazol-4-yl]methanone | 1H NMR (600 MHz, CHLOROFORM-d) *δ* ppm 1.09 (d, J=6.19 Hz, 3 H) 1.38 - 1.46 (m, 1H) 1.65 - 1.74 (m, 1 H) 1.74 - 1.83 (m, 1 H) 1.88 - 1.96 (m, 1 H) 1.97 - 2.06 (m, 2 H) 2.12 (q, J=8.67 Hz, 1 H) 2.18 - 2.24 (m, 1 H) 2.26 - 2.34 (m, 1 H) 2.39 - 2.46 (m, 2 H) 2.95 - 3.02 (m, 1 H) 3.18 (td, J=8.57, 2.68 Hz, 1 H) 4.05 - 4.12 (m, 2 H) 4.26 (t, J=7.84 Hz, 2 H) 4.81 (t, J=7.84 Hz, 2 H) 7.01 - 7.06 (m, 2 H) 7.59 - 7.65 (m, 2 H) 8.43 (s, 1 H); MS (ESI pos.) m/z : 370 [M+H]+ |

### (Example 3)

### Preparation of 1-{4-[(1-cyclobutylpiperidin-4-yl)oxy]phenyl}-1H-1,2,3-triazole-4-carboxylic acid hydrochloride (Compound No. 17)

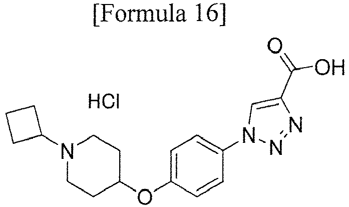

A mixture of the ethyl 1-{4-[(1-cyclobutylpiperidin-4-yl)oxy]phenyl}-1H-1,2,3-triazole-4-carboxylate (0.27 g) prepared in Example 1 and concentrated hydrochloric acid (1.0 mL) was stirred at 100°C for 5 hours. After the reaction mixture was concentrated under reduced pressure, tetrahydrofuran (1.0 mL) was added to the residue, the mixture was stirred at 0°C, and then the precipitate was collected by filtration to give the titled compound as a colorless solid (0.27 g).
1H NMR (600 MHz, DMSO-d₆) δ ppm 1.62 - 1.83 (m, 2 H) 1.87 - 2.00 (m, 1 H) 2.02 - 2.11 (m, 1 H) 2.12 - 2.22 (m, 3 H) 2.26 (d, J=13.21 Hz, 1 H) 2.30 - 2.42 (m, 2 H) 2.80 - 3.01 (m, 2H)3.19-3.28 (m, 1 H)3.56-3.67(m, 1 H) 3.69 - 3.80 (m, 1 H) 4.62 - 4.92 (m, 1 H) 7.17 - 7.28 (m, 2 H) 7.82 - 7.97 (m, 2 H) 9.30 (s, 1 H) 10.85 (br. s., 1 H) 13.29 (br. s., 1 H); MS (ESI pos.) m/z : 343 [M+H]⁺

### (Example 4)

### Preparation of azetidin-1-yl(1-{4-[(1-cyclobutylpiperidin-4-yl)oxy]phenyl} -1H-1,2,3-triazol-4-yl)methanone (Compound No. 18)

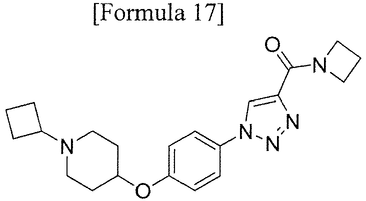

The 1-{4-[(1-cyclobutylpiperidin-4-yl)oxy]phenyl}-1H-1,2,3-triazole-4-carboxylic acid (0.10 g) prepared in Example 3, azetidine hydrochloride (0.037 g), 1-{3-(dimethylamino)propyl}-3-ethylcarbodiimide hydrochloride (0.076 g), 1-hydroxybenzotriazole hydrate (0.061 g) and triethylamine (0.11 mL) were added to N,N-dimethylformamide (1.0 mL) to prepare a suspension, followed by stirring at room temperature for 16 hours. To the reaction mixture, saturated aqueous sodium hydrogencarbonate was added, and the mixture was extracted with chloroform. The organic layer was dried over sodium sulfate and then concentrated under reduced pressure. The resulting residue was purified by silica gel column chromatography (eluent: chloroform/methanol = 100/0 - 95/5). The resulting solid was washed with diisopropyl ether to give the titled compound as a colorless solid (0.058 g).
1H NMR (600 MHz, CHLOROFORM-d) δ ppm 1.64 - 1.77 (m, 2 H) 1.88 (d, J=9.50 Hz, 4 H) 1.96 - 2.26 (m, 6 H) 2.39 - 2.47 (m, 2 H) 2.64 (br. s., 2 H) 2.75 (br. s., 1 H) 4.22 - 4.29 (m, 2 H) 4.39 (br. s., 1 H) 4.80 (t, J=7.64 Hz, 2 H) 7.00 - 7.05 (m, 2 H) 7.62 (d, J=9.08 Hz, 2 H) 8.42 (s, 1 H); MS (ESI pos.) m/z : 382 [M+H]⁺

### (Example 5)

### Preparation of 1-{4-[(1-cyclobutylpiperidin-4-yl)oxylphenyl}-N-methyl-1H-1,2,3-triazole-4-carboxamide (Compound No. 19)

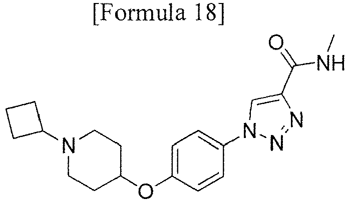

The titled compound was prepared as a colorless solid by repeating the procedure of Example 4, except that the azetidine hydrochloride was replaced by methylamine hydrochloride.
1H NMR (600 MHz, CHLOROFORM-d) δ ppm 1.62 - 1.78 (m, 2 H) 1.81 - 1.94 (m, 4 H) 1.99 - 2.10 (m, 4 H) 2.12 - 2.24 (m, 2 H) 2.58 - 2.69 (m, 2 H) 2.70 - 2.80 (m, 1 H) 3.05 (d, J=5.37 Hz, 3 H) 4.39 (br. s., 1 H) 7.02 - 7.06 (m, 2 H) 7.18 (br. s., 1 H) 7.61 (d, J=9.08 Hz, 2 H) 8.39 (s, 1 H); MS (ESI pos.) m/z : 356 [M+H]⁺

### (Example 6)

### Preparation of 1-{4-[(1-cyclobutylpiperidin-4-yl)oxy]phenyl}-1H-1,2,3-triazole-4-carboxamide (Compound No. 20)

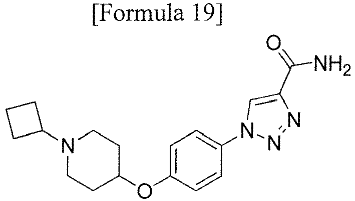

The titled compound was prepared as a colorless solid by repeating the procedure of Example 4, except that the azetidine hydrochloride was replaced by ammonia water.
1HNMR (600 MHz, CHLOROFORM-d) δ ppm 1.62 - 2.27 (m, 12 H) 2.56 - 2.82 (m, 3 H) 4.40 (br. s., 1 H) 5.60 (br. s., 1 H) 7.00 - 7.12 (m, 3 H) 7.59 - 7.65 (m, 2 H) 8.42 (s, 1 H); MS (ESI pos.) m/z : 342 [M+H]⁺

### (Example 7)

### Preparation of 1-{4-[(1-cyclobutylpiperidin-4-yl)oxy]phenyl}-1H-1,2,3-triazole-4-carbonitrile hydrochloride (Compound No. 21)

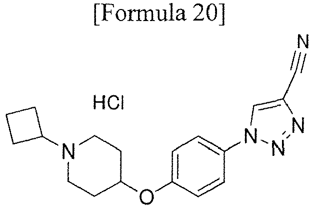

The 1-{4-[(1-cyclobutylpiperidin-4-yl)oxy]phenyl}-1H-1,2,3-triazole-4-carboxamide (0.1 g) prepared in Example 6 was added to N,N-dimethylformamide (1.0 mL) to prepare a solution, thionyl chloride (0.348 g) was added dropwise thereto while cooling in ice, and the mixture was stirred for 3 hours while heating to room temperature. Water and ethanol were added to the reaction mixture, and the precipitated crystal was collected by filtration. The resulting crude crystal was recrystallized from ethanol to give the titled compound as a coloreless crystal (0.060 g).
1H NMR (600 MHz, DMSO-d₆) δ ppm 1.63 - 1.82 (m, 2 H) 1.82 - 1.95 (m, 1 H) 2.04 - 2.14 (m, 2 H) 2.14 - 2.23 (m, 2 H) 2.23 - 2.36 (m, 2 H) 2.82 - 3.02 (m, 2 H) 3.19 - 3.49 (m, 2 H) 3.58 - 3.81 (m, 1 H) 4.64 - 4.94 (m, 1 H) 7.21 - 7.32 (m, 2 H) 7.78 - 7.91 (m, 2 H) 9.65 (s, 1 H) 10.42 (br. s., 1 H); MS (ESI pos.) m/z : 324 [M+H]⁺

### (Example 8)

### Preparation of (1-{4-[(1-cyclobutylpiperidin-4-yl)oxy]phenyl}-1H-1,2,3-triazol-4-yl)methanol (Compound No. 22)

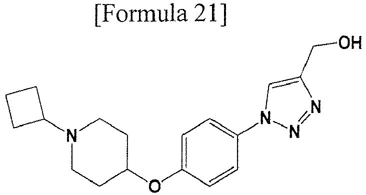

The titled compound was prepared as a colorless solid by repeating the procedure of Example 1, except that the ethyl propiolate was replaced by 2-propyn-1-ol.
1H NMR (600 MHz, CHLOROFORM-d) δ ppm 1.44 - 2.27 (m, 12 H) 2.61 (d, J=12.39 Hz, 2H)2.71 2.83 (m, 1 H) 4.30 - 4.43 (m, 1 H) 4.87 (d, J=6.19 Hz, 2 H) 6.93 - 7.05 (m, 2 H) 7.48 - 7.64 (m, 2 H) 7.87 (s, 1 H); MS (ESI pos.) m/z : 329 [M+H]+

### (Example 9)

### Preparation of 1-cyclobutyl-4-[4-(1H-1,2,3-triazol-1-yl)phenoxy]piperidine (Compound No. 23)

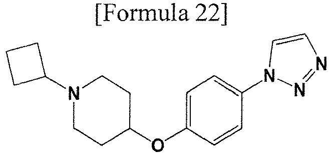

A mixture of 1-cyclobutyl-4-(4-iodophenoxy)piperidine (0.1 g, which can be synthesized according to the method described in WO2008072703), sodium azide (0.033 g), N,N'-dimethylethylenediamine (4.9 mg), copper iodide (5.3 mg), sodium ascorbate (2.3 mg), ethanol (0.9 mL) and water (0.1 mL) was stirred at 70°C for 2 hours. The reaction mixture was left to cool to room temperature, potassium carbonate (58 mg) and ethynyltrimethylsilane (33 mg) were added, and the mixture was stirred at 70°C for 4 hours. The reaction mixture was left to cool to room temperature, ammonia water was added, the mixture was extracted with ethyl acetate, and then the resulting organic layer was washed with ammonia water and brine sequentially. The organic layer was concentrated under reduced pressure. The resulting residue was purified by silica gel column chromatography (NH form silica gel; eluent: chloroform). The resulting solid was washed with diisopropyl ether to give the titled compound as a colorless solid (0.050 g).
1H NMR (600 MHz, CHLOROFORM-d) δ ppm 1.46 - 2.41 (m, 11 H) 2.53 - 2.91 (m, 4 H) 4.30 - 4.51 (m, 1 H) 7.00 - 7.05 (m, 2 H) 7.62 (d, J=9.08 Hz, 2 H) 7.83 (d, J=0.83 Hz, 1 H) 7.90 (d, J=0.83 Hz, 1 H); MS (ESI pos.) m/z : 299 [M+H]+

### (Example 10)

### Preparation of ethyl 1-(4-{3-[(2R)-2-methylpyrrolidin-1-yl]propoxy}phenyl)-1H-1,2,3-triazole-4-carboxylate (Compound No. 24)

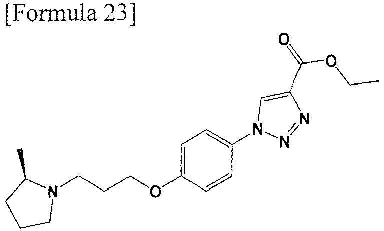

The titled compound was prepared as a yellow solid by repeating the procedure of Example 1, except that the 1-cyclobutyl-4-(4-iodophenoxy)piperidine was replaced by (2R)-1-[3-(4-iodophenoxy)propyl]-2-methylpyrrolidine (which can be synthesized according to the method described in WO2009063953).
1H NMR (600 MHz, CHLOROFORM-d) δ ppm 0.91 - 1.02 (m, 3 H) 1.25 - 1.37 (m, 4 H) 1.52 - 2.24 (m, 8 H) 2.87 (dt, J=11.97, 8.05 Hz, 1 H) 3.06 (td, J=8.46, 2.48 Hz, 1 H) 3.91 - 4.06 (m, 2 H) 4.35 (q, J=7.02 Hz, 2 H) 6.86 - 6.96 (m, 2 H) 7.45 - 7.57 (m, 2 H) 8.29 (s, 1 H); MS (ESI pos.) m/z : 359 [M+H]+

### (Example 11)

### Preparation of (1-{4-[(1-cyclobutylpiperidin-4-yl)oxy]phenyl}-1H-1,2,3-triazol-4-yl)(morpholin-4-yl)methanone (Compound No. 25)

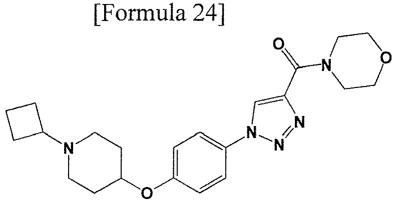

The titled compound was prepared as a colorless solid by repeating the procedure of Example 4, except that the azetidine hydrochloride was replaced by morpholine.
1H NMR (600 MHz, CHLOROFORM-d) δ ppm 1.57 - 2.32 (m, 12 H) 2.58 - 2.83 (m, 3 H) 3.77 - 3.86 (m, 6 H) 4.35 - 4.46 (m, 3 H) 7.01 - 7.07 (m, 2 H) 7.60 - 7.65 (m, 2 H) 8.43 (s, 1 H); MS (ESI pos.) m/z : 412 [M+H]+

### (Example 12)

### Preparation of 1-{4-[(1-cyclobutylpiperidin-4-yl)oxy]phenyl}-N,N-dimethyl-1H-1,2,3-triazole-4-carboxamide (Compound No. 26)

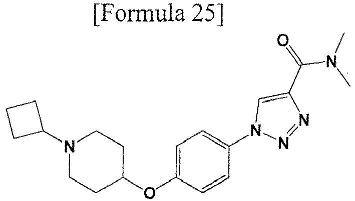

The titled compound was prepared as a colorless solid by repeating the procedure of Example 4, except that the azetidine hydrochloride was replaced by dimethylamine (2.0 M THF solution).
1H NMR (600 MHz, CHLOROFORM-d) δ ppm 1.63 - 2.29 (m, 12 H) 2.57 - 2.80 (m, 3 H) 3.16 (s, 3 H) 3.60 (s, 3 H) 4.34 - 4.43 (m, 1 H) 7.01 - 7.05 (m, 2 H) 7.63 (d, J=9.08 Hz, 2 H) 8.41 (s, 1 H); MS (ESI pos.) m/z : 370 [M+H]+

### (Example 13)

### Preparation of ethyl 1-{4-[(1-tert-butylpiperidin-4-yl)oxy]phenyl}-1H-1,2,3-triazole-4-carboxylate (Compound No. 27)

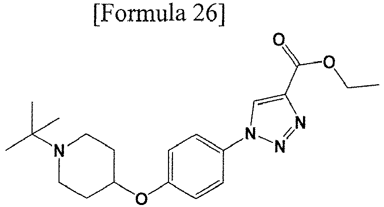

The titled compound was prepared as a colorless solid by repeating the procedure of Example 1, except that the 1-cyclobutyl-4-(4-iodophenoxy)piperidine was replaced by 1-(tert-butyl)-4-(4-iodophenoxy)piperidine (which can be synthesized according to the method described in WO2008072724).
1H NMR (600 MHz, CHLOROFORM-d) δ ppm 1.03 - 1.19 (m, 9 H) 1.45 (t, J=7.22 Hz, 3 H) 1.86 (br. s., 2 H) 2.06 (d, J=6.61 Hz, 2 H) 2.46 (br. s., 2 H) 2.90 (br. s., 2 H) 4.37 (br. s., 1 H) 4.48 (q, J=7.16 Hz, 2 H) 6.95 - 7.11 (m, 2 H) 7.55 - 7.69 (m, 2 H) 8.42 (s, 1 H); MS (ESI pos.) m/z : 373 [M+H]+

### (Example 14)

Preparation of (1-{4-[(1-tert-butylpiperidin-4-yl)oxy]phenyl}-1H-1,2,3-triazol-4-yl)(pyrrolidin-1-yl)methanone (Compound No. 28)

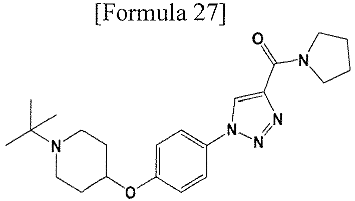

The titled compound was prepared as a colorless solid by repeating the procedure of Example 2, except that the ethyl 1-{4-[(1-cyclobutylpiperidin-4-yl)oxy]phenyl}-1H-1,2,3-triazole-4-carboxylate was replaced by the ethyl 1-[4-(1-tert-butylpiperidin-4-yl)oxyphenyl]triazole-4-carboxylate prepared in Example 13.
1H NMR (600 MHz, DMSO-d6) δ ppm 1.00 (s, 9 H) 1.49 - 1.63 (m, 2 H) 1.75 - 1.84 (m, 2 H) 1.87 - 1.98 (m, 4 H) 2.25 - 2.34 (m, 2 H) 2.76 - 2.84 (m, 2 H) 3.46 - 3.51 (m, 2 H) 3.87 - 3.92 (m, 2 H) 4.33 - 4.44 (m, 1 H) 7.11 (d, J=9.08 Hz, 2 H) 7.81 (d, J=9.08 Hz, 2 H) 9.11 (s, 1 H); MS (ESI pos.) m/z : 398 [M+H]+

### (Example 15)

### Preparation of azetidin-1-yl(1-{4-[(1-tert-butylpiperidin-4-yl)oxy]phenyl}-1H-1,2,3-triazol-4-yl)methanone (Compound No. 29)

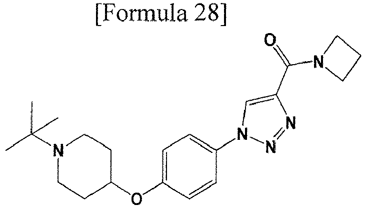

The titled compound was prepared as a colorless solid by repeating the procedure of Example 14, except that the pyrrolidine was replaced by azetidine.
1H NMR (600 MHz, DMSO-d6) δ ppm 0.99 (s, 9 H) 1.48 - 1.61 (m, 2 H) 1.86 - 1.97 (m, 2 H) 2.29 (d, J=7.43 Hz, 4 H) 2.73 - 2.85 (m, 2 H) 4.01 - 4.05 (m, 2 H) 4.31 - 4.41 (m, 1 H) 4.53 - 4.58 (m, 2 H) 7.10 (d, J=9.08 Hz, 2 H) 7.79 (d, J=9.08 Hz, 2 H) 9.12 (s, 1 H); MS (ESI pos.) m/z : 384 [M+H]+

### (Example 16)

### Preparation of 1-{4-[(1-tert-butylpipendin-4-yl)oxy]phenyl}-N-methyl-1H-1,2,3-triazole-4-carboxamide (Compound No. 30)

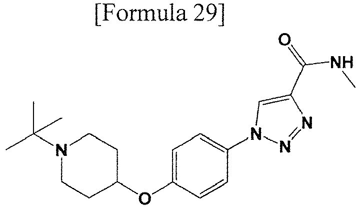

The titled compound was prepared as a colorless solid by repeating the procedure of Example 16, except that the pyrrolidine was replaced by methylamine (9.8 M methanol solution).
1H NMR (600 MHz, DMSO-d6) δ ppm 0.96 (s, 9 H) 1.52 (d, J=9.08 Hz, 2 H) 1.89 (br. s., 2 H) 2.27 (br. s., 2 H) 2.73 (d, J=4.54 Hz, 3 H) 2.75 - 2.82 (m, 2 H) 4.30 - 4.41 (m, 1 H) 7.07 (d, J=9.08 Hz, 2 H) 7.76 (d, J=9.08 Hz, 2 H) 8.50 (d, J=4.54 Hz, 1 H) 9.07 (s, 1 H); MS (ESI pos.) m/z : 358 [M+H]+

### (Example 17)

### Preparation of 1-{4-[(1-tert-butylpiperidin-4-yl)oxy]phenyl}-1H-1,2,3-triazole-4-carboxamide (Compound No. 31)

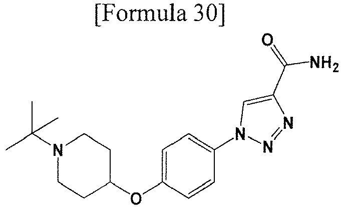

A mixture of the ethyl 1-[4-(1-tert-butylpiperidin-4-yl)oxyphenyl]triazole-4-carboxylate (0.30 g) prepared in Example 13, 6 N aqueous sodium hydroxide (0.40 mL) and ethanol (2.0 mL) was stirred at 60°C for 3 hours. To the reaction mixture, saturated aqueous ammonium chloride was added, and the mixture was concentrated under reduced pressure. To the residue, 1-{3-(dimethylamino)propyl}-3-ethylcarbodiimide hydrochloride (0.31 g), 1-hydroxybenzotriazole hydrate (0.25 g) and N,N-dimethylformamide (2.0 mL) were added to prepare a suspension, followed by stirring at room temperature for 16 hours. Twenty-eight percent aqueous ammonia (0.2 mL) was added and the mixture was stirred at room temperature for 16 hours. Water was added to the reaction mixture and the precipitate was collected by filtration. This crude crystal was recrystallized from ethanol-water to give the titled compound as a colorless solid (0.086 g).
1H NMR (600 MHz, DMSO-d6) δ ppm 1.00 (s, 9 H) 1.50 - 1.62 (m, 2 H) 1.89 - 1.98 (m, 2 H) 2.24 - 2.32 (m, 2 H) 2.74 - 2.85 (m, 2 H) 4.32 - 4.45 (m, 1 H) 7.11 (d, J=9.50 Hz, 2 H) 7.56 (br.s, 1/2 H) 7.78 (d, J=9.08 Hz, 2 H) 7.93 (br.s, 1/2 H) 9.08 (s, 1 H); MS (ESI pos.) m/z : 344 [M+H]+

### (Example 18)

### Preparation of 1-{4-[(1-tert-butylpiperidin-4-yl)oxy]phenyl}-1H-1,2,3-triazole-4-carbonitrile (Compound No. 32)

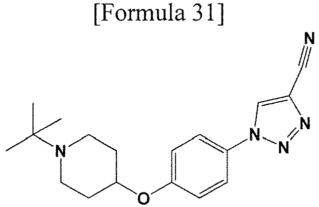

The titled compound was prepared as a colorless solid by repeating the procedure of Example 7, except that the 1-{4-[(1-cyclobutylpiperidin-4-yl)oxy]phenyl}-1H-1,2,3-triazole-4-carboxamide was replaced by 1-[4-(1-tert-butylpiperidin-4-yl)oxyphenyl]triazole-4-carboxamide.
1H NMR (600 MHz, CHLOROFORM-d) δ ppm 1.09 (br. s., 9 H) 1.83 (br. s., 2 H) 2.02 (br. s., 2 H) 2.43 (br. s., 2 H) 2.87 (br. s., 2 H) 4.27 - 4.42 (m, 1 H) 6.99 - 7.09 (m, 2 H) 7.58 (d, J=9.08 Hz, 2 H) 8.30 (s, 1 H); MS (ESI pos.) m/z : 326 [M+H]+

### (Example 19)

### Preparation of ethyl 1-{4-[(1-cyclobutylpiperidin-4-yl)oxy]phenyl}-1H-1,2,4-triazole-3-carboxylate (Compound No. 33)

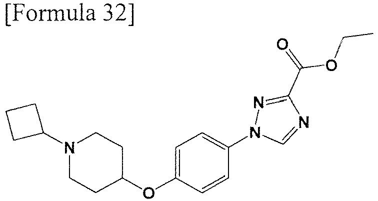

1-Cyclobutyl-4-(4-nitrophenoxy)piperidine (5.5 g, which can be synthesized according to the method described in the Journal of Medicinal Chemistry, 2008, Vol. 51, p. 6889), 5% palladium-carbon (0.55 g) and methanol (55 mL) were mixed under a hydrogen atmosphere to prepare a suspension, followed by stirring at room temperature for 4 hours. The reaction mixture was filtered through Celite (registered trademark). The filtrate was concentrated under reduced pressure. 1 N aqueous hydrochloric acid (4.9 mL) was added to the residue to prepare a solution. To this solution, a mixture of sodium nitrite (125 mg) and water (0.52 mL) was added dropwise while cooling in ice, and the mixture was stirred for 20 minutes. This reaction mixture was added dropwise to a mixture of ethyl cyanoacetate (1.8 g), sodium acetate (12.1 g), methanol (87.0 mL) and water (11.0 mL) while cooling in ice, and the mixture was stirred for 1 hour. Water was added to the reaction mixture, and the mixture was concentrated under reduced pressure and then extracted with chloroform. The organic layer was concentrated under reduced pressure. The resulting residue was purified by silica gel column chromatography (NH form silica gel; eluent: hexane/ethyl acetate = 88/12 - 0/100) to give the titled compound as a brown oil (1.7 g).
1H NMR (600 MHz, CHLOROFORM-d) δ ppm 1.40 (t, J=7.22 Hz, 3 H) 1.57 - 1.70 (m, 2 H) 1.74 - 1.88 (m, 4 H) 1.91 - 2.04 (m, 4 H) 2.12 (br. s., 2 H) 2.57 (br. s., 2 H) 2.69 (t, J=7.64 Hz, 1 H) 4.32 (br. s., 1 H) 4.44 - 4.49 (m, 2 H) 6.91 - 7.01 (m, 2 H) 7.52 - 7.62 (m, 2 H) 8.43 (s, 1 H); MS (ESI pos.) m/z : 371 [M+H]+

### (Example 20)

### Preparation of (1-{4-[(1-cyclobutylpiperidin-4-yl)oxy]phenyl}-1H-1,2,4-triazol-3-yl)(pyrrolidin-1-yl)methanone (Compound No. 34)

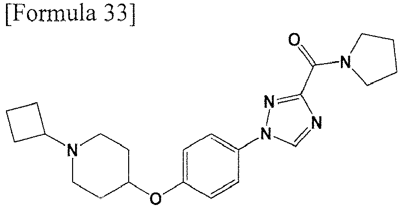

The titled compound was prepared as a colorless solid by repeating the procedure of Example 2, except that the ethyl 1-{4-[(1-cyclobutylpiperidin-4-yl)oxy]phenyl}-1H-1,2,3-triazole-4-carboxylate was replaced by the 1-{4-[(1-cyclobutylpiperidin-4-yl)oxy]phenyl}-1H-1,2,4-triazole-3-carboxylate prepared in Example 19.
1 H NMR (600 MHz, CHLOROFORM-d) δ ppm 1.62 - 1.74 (m, 2 H) 1.79 - 2.08 (m, 12 H) 2.16 (d, J=2.89 Hz, 2 H) 2.62 (br. s., 2 H) 2.69 - 2.78 (m, 1 H) 3.72 (t, J=6.81 Hz, 2 H) 3.92 (t, J=6.61 Hz, 2 H) 4.35 (br. s., 1 H) 6.95 - 7.05 (m, 2 H) 7.51 - 7.61 (m, 2 H) 8.44 (s, 1 H); MS (ESI pos.) m/z : 396 [M+H]+

### (Example 21)

### Preparation of azetidin-1-yl(1-{4-[(1-cyclobutylpiperidin-4-yl)oxy]phenyl}-1H-1,2,4-triazol-3-yl)methanone (Compound No. 35)

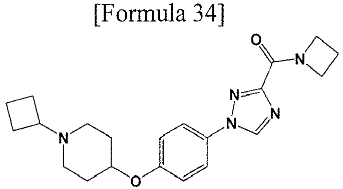

The titled compound was prepared as a colorless solid by repeating the procedure of Example 20, except that the pyrrolidine was replaced by azetidine.
1H NMR (600 MHz, CHLOROFORM-d) δ ppm 1.53 - 1.77 (m, 2 H) 1.81 - 1.94 (m, 4 H) 1.97 - 2.08 (m, 4 H) 2.17 (br. s., 2 H) 2.30 - 2.41 (m, 2 H) 2.62 (br. s., 2 H) 2.74 (t, J=7.84 Hz, 1 H) 4.27 (t, J=7.84 Hz, 2 H) 4.36 (br. s., 1 H) 4.66 (t, J=7.64 Hz, 2 H) 6.99 (d, J=8.26 Hz, 2 H) 7.57 (d, J=9.08 Hz, 2 H) 8.43 (d, J=0.83 Hz, 1 H); MS (ESI pos.) m/z : 382 [M+H]+

### (Example 22)

### Preparation of (1-{4-[(1-cyclobutylpiperidin-4-yl)oxy]phenyl}-1H-1,2,4-triazol-3-yl)(piperidin-1-yl)methanone (Compound No. 36)

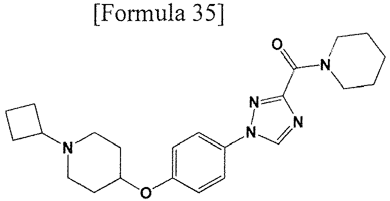

The titled compound was prepared as a colorless solid by repeating the procedure of Example 20, except that the pyrrolidine was replaced by piperidine.
1H NMR (600 MHz, CHLOROFORM-d) δ ppm 1.55 - 1.73 (m, 8 H) 1.79 - 1.94 (m, 4 H) 1.97 - 2.08 (m, 4 H) 2.16 (br. s., 2 H) 2.62 (br. s., 2 H) 2.74 (t, J=7.84 Hz, 1 H) 3.68 (d, J=5.78 Hz, 2 H) 3.75 (t, J=4.95 Hz, 2 H) 4.35 (br. s., 1 H) 6.91 - 7.05 (m, 2 H) 7.49 - 7.61 (m, 2 H) 8.43 (s, 1 H); MS (ESI pos.) m/z : 410 [M+H]+

### (Example 23)

### Preparation of N-tert-butyl-1-{4-[(1-cyclobutylpiperidin-4-yl)oxy]phenyl}-1H-1,2,4-triazole-3-carboxamide (Compound No. 37)

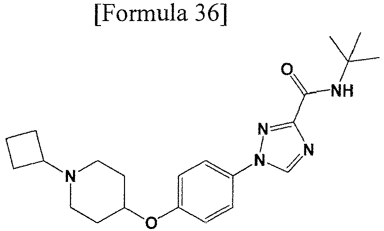

The titled compound was prepared as a colorless solid by repeating the procedure of Example 20, except that the pyrrolidine was replaced by tert-butylamine.
1H NMR (600 MHz, CHLOROFORM-d) δ ppm 1.42 (s, 9 H) 1.54 - 1.67 (m, 2 H) 1.70 - 1.86 (m, 4 H) 1.89 - 2.01 (m, 4 H) 2.06 - 2.13 (m, 2 H) 2.46 - 2.58 (m, 2 H) 2.61 - 2.71 (m, 1 H) 4.20 - 4.34 (m, 1 H) 6.92 (d, J=9.08 Hz, 3 H) 7.52 (d, J=9.08 Hz, 2 H) 8.31 (s, 1 H); MS (ESI pos.) m/z : 398 [M+H]+

### (Example 24)

### Preparation of 1-{4-[(1-cyclobutylpiperidin-4-yl)oxy]phenyl}-1H-1,2,4-triazole-3-carboxylic acid hydrochloride (Compound No. 38)

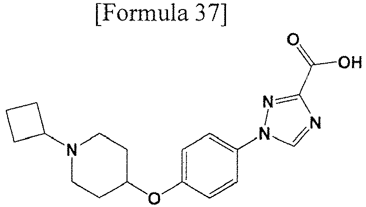

A mixture of the ethyl 1-{4-[(1-cyclobutylpiperidin-4-yl)oxy]phenyl}-1H-1,2,4-triazole-3-carboxylate (0.60 g) prepared in Example 19 and concentrated hydrochloric acid (6.0 mL) was stirred at 100°C for 5 hours. The reaction mixture was left to cool to room temperature and then concentrated under reduced pressure. Tetrahydrofuran (2.0 mL) was added to the residue and the mixture was stirred for 30 minutes while cooling in ice. The precipitate was collected by filtration to give the titled compound as a colorless solid.
1H NMR (600 MHz, CHLOROFORM-d) δ ppm 1.63 - 1.74 (m, 2 H) 1.75 - 1.82 (m, 2 H) 1.84 - 1.92 (m, 2 H) 1.92 - 1.99 (m, 2 H) 2.00 - 2.17 (m, 4 H) 2.72 (t, J=7.79 Hz, 1 H) 2.77 - 2.84 (m, 2 H) 2.98 - 3.05 (m, 2 H) 4.22 (br. s., 1 H) 6.31 (d, J=9.17 Hz, 1 H) 6.60 (dd, J=8.71, 2.75 Hz, 1 H) 6.78 (d, J=2.75 Hz, 1 H) 6.86 (d, J=8.71 Hz, 1 H) 7.44 (dd, J=8.71, 2.75 Hz, 1 H) 8.04 (d, J=2.75 Hz, 1 H); MS (ESI pos.) m/z : 343 [M+H]+

### (Example 25)

### Preparation of 1-{4-[(1-cyclobutylpiperidin-4-yl)oxy]phenyl}-1H-1,2,4-triazole-3-carboxamide (Compound No. 39)

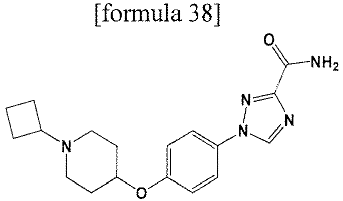

The 1-{4-[(1-cyclobutylpiperidin-4-yl)oxy]phenyl}-1H-1,2,4-triazole-3 -carboxylic acid hydrochloride (0.30 g) prepared in Example 24, 1-{3-(dimethy-lamino)propyl}-3-ethylcarbodiimide hydrochloride (0.23 g), 1-hydroxybenzotriazole hydrate (0.18 g), triethylamine (0.16 g) and N,N-dimethylformamide (0.6 mL) were added to prepare a suspension, followed by stirring at room temperature for 30 minutes. To the reaction mixture, ammonia (0.23 mL, 7 M methanol solution) was added, and the mixture was stirred at room temperature for 16 hours. To the reaction mixture, water was added, and the mixture was extracted with chloroform. The organic layer was dried over anhydrous magnesium sulfate and then concentrated under reduced pressure. The residue was purified by silica gel column chromatography (NH form silica gel; eluent: ethyl acetate) to give the titled compound as a colorless solid (0.093 g).
1H NMR (600 MHz, CHLOROFORM-d) δ ppm 1.54 - 1.66 (m, 2 H) 1.70 - 1.85 (m, 4 H) 1.96 (br. s., 4 H) 2.05 - 2.16 (m, 2 H) 2.48 - 2.59 (m, 2 H) 2.62 - 2.72 (m, 1 H) 4.23 - 4.34 (m, 1 H) 5.51 - 5.62 (m, 1 H) 6.86 - 6.96 (m, 3 H) 7.52 (d, J=9.08 Hz, 2 H) 8.37 (s, 1 H); MS (ESI pos.) m/z : 342 [M+H]+

### (Example 26)

### Preparation of 1-(1-{4-[(1-cyclobutylpiperidin-4-yl)oxy]phenyl}-1H-1,2,4-triazol-3-yl)ethanone hydrochloride (Compound No. 40)

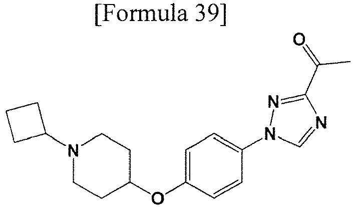

The 1-{4-[(1-cyclobutylpiperidin-4-yl)oxy]phenyl}-1H-1,2,4-triazole-3 -carboxylic acid hydrochloride (0.40 g) prepared in Example 24, 1-{3-(dimethylamino)propyl}-3-ethylcarbodiimide hydrochloride (0.24 g), 1-hydroxybenzotriazole hydrate (0.16 g), triethylamine (0.21 g), N,O-dimethylhydroxy-lamine hydrochloride (0.12 g) and N,N-dimethylformamide (5.0 mL) were added to prepare a suspension, followed by stirring at room temperature for 16 hours. To the reaction mixture, saturated aqueous sodium hydrogencarbonate was added, and the mixture was extracted with chloroform. The organic layer was washed with saturated aqueous sodium hydrogencarbonate and brine sequentially and then concentrated under reduced pressure. The residue was purified by silica gel column chromatography (eluent: chloroform/methanol = 10/0 - 9/1) to give 1-{4-[(1-cyclobutylpiperidin-4-yl)oxy]phenyl}-N-methoxy-N-methyl-1H-1,2,4-triazole-3-carboxamide as a colorless solid (0.33 g). MS (ESI pos.) m/z : 386 [M+H]⁺

To a solution of 1-{4-[(1-cyclobutylpiperidin-4-yl)oxy]phenyl}-N-methoxy-N-methyl-1H-1,2,4-triazole-3-carboxamide (0.10 g) in tetrahydrofuran (3.0 mL), methyllithium (0.52 mL, 1.0 M diethyl ether solution) was added dropwise at -78°C, and the mixture was stirred for 2 hours, then heated to room temperature and stirred for 1 hour. To the reaction mixture, saturated aqueous ammonium chloride was added, and the mixture was extracted with chloroform and then concentrated under reduced pressure. The residue was purified by silica gel column chromatography (eluent: chloroform/methanol = 10/0 - 9/1). After the resulting amorphous solid was dissolved in ethyl acetate (2.0 mL), hydrochloric acid (1.0 mL, 4 M ethyl acetate solution) was added dropwise and the mixture was stirred at room temperature for 10 minutes. The reaction mixture was concentrated under reduced pressure to give the titled compound as a colorless solid (12.0 mg).
1H NMR (600 MHz, DMSO-d6) δ ppm 1.65 - 1.80 (m, 2 H) 2.04 - 2.40 (m, 8 H) 2.62 (s, 3 H) 2.83 - 2.99 (m, 3 H) 3.20 - 3.26 (m, 1 H) 3.39 - 3.45 (m, 1 H) 3.71 - 3.80 (m, 1 H) 7.23 (dd, J=18.58, 9.08 Hz, 2 H) 7.84 (dd, J=11.15, 9.08 Hz, 2 H) 9.34 (s, 1 H); MS (ESI pos.) m/z : 341 [M+H]+

### (Example 27)

### Preparation of 1-{4-[(1-cyclobutylpiperidin-4-yl)oxy]phenyl}-1H-1,2,4-triazole-3-carbonitrile (Compound No. 41)

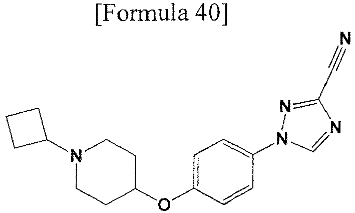

To an N,N-dimethylformamide (0.6 mL) solution of the 1-{4-[(1-cyclobutylpiperidin-4-yl)oxy]phenyl}-1H-1,2,4-triazole-3-carboxamide (0.060 g) prepared in Example 25, thionyl chloride (0.21 g) was added while cooling in ice, and the mixture was stirred at room temperature for 2 hours. To the reaction mixture, saturated aqueous sodium hydrogencarbonate was added, and the mixture was extracted with chloroform and then concentrated under reduced pressure. The residue was purified by preparative thin layer chromatography (NH form silica gel; eluent: hexane/ethyl acetate = 1/1) to give the titled compound as a colorless solid (0.025 g).
1H NMR (600 MHz, CHLOROFORM-d) δ ppm 1.61-1.74 (m, 2 H) 1.77 - 1.91 (m, 4 H) 1.95 - 2.06 (m, 4 H) 2.07 - 2.21 (m, 2 H) 2.54 - 2.63 (m, 2 H) 2.67 - 2.77 (m, 1 H) 4.32 - 4.40 (m, 1 H) 7.00 (d, J=9.08 Hz, 2 H) 7.52 (d, J=9.08 Hz, 2 H) 8.47 (s, 1 H); MS (ESI pos.) m/z : 324 [M+H]+

### (Test 1: Rat H3 receptor binding test)

The frontal cortex dissected from rats was homogenized with a Teflon (registered trademark) homogenizer in a 50 mM Tris-HCl buffer solution (pH 7.4) containing a protease inhibitor (Complete EDTA-free; Roche Diagnostics) and 5 mM EDTA. The homogenate was centrifuged at 48,000×g for 15 minutes. The supernatant was removed and the pellet was suspended in a 50 mM Tris-HCl buffer solution (pH 7.4) containing 5 mM EDTA and centrifuged at 48,000xg for 15 minutes. The supernatant was removed and the pellet was suspended in a 50 mM Tris-HCl buffer solution (pH 7.4) containing 5 mM EDTA to give a membrane fraction. The membrane fraction (the protein content in the final reaction mixture: 75 µg), N-α-methyl[³H]histamine (PerkinElmer; final concentration: 0.75 nM) and a test drug were mixed and subjected to reaction at room temperature for an hour. After the end of the reaction, the reaction mixture was suction filtered through a 96-well GF/C filter plate pretreated with 0.3% polyethyleneimine; the filters were then washed five times with a 50 mM Tris-HCl buffer solution (pH 7.4) containing 5 mM EDTA. After the washing, the filters were dried and a scintillator was added to measure the residual radioactivity on the filter with TopCount (PerkinElmer).

The residual radioactivity in the presence of 10 µM thioperamide was taken as indicative of nonspecific binding and the difference from the residual radioactivity in the absence of thioperamide was taken as indicative of specific binding. Each of the test drugs was dissolved and diluted in DMSO at varying concentrations to plot a dose-response curve from the corresponding residual radioactivities; the concentration of test drug that inhibited the specific binding by 50% (IC₅₀) was determined from this curve. The IC₅₀ values of the example compounds are shown in Table 3 below.

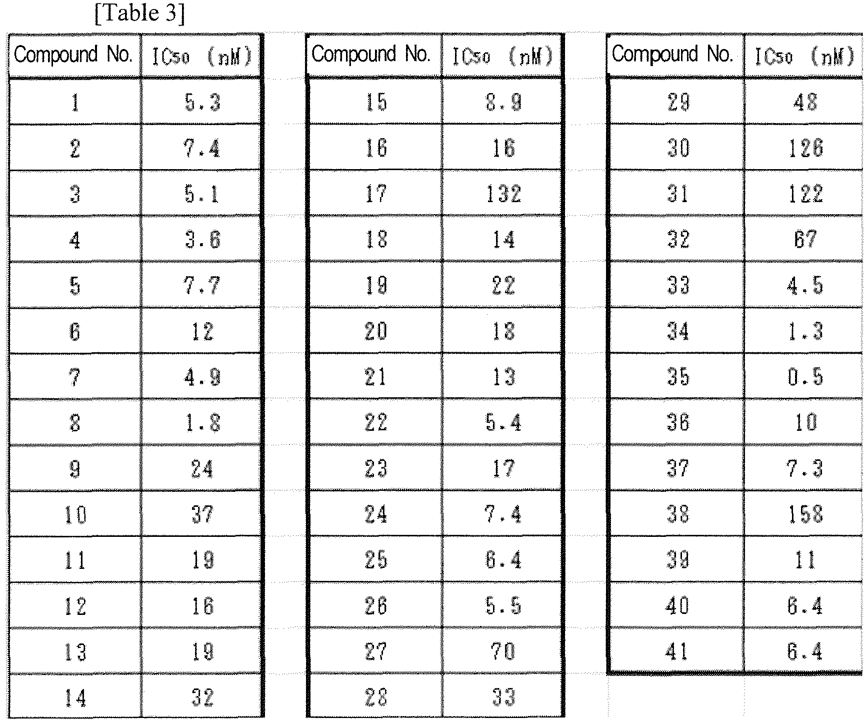

### (Test 2: [³⁵S]GTP-γ-S binding test)

The frontal cortex dissected from rats was homogenized with a Teflon (registered trademark) homogenizer in a 30 mM Tris-HCl buffer solution (pH 7.4) containing 2.5 mM calcium chloride dihydrate. The homogenate was centrifuged at 48,000×g for 15 minutes. The supernatant was removed and the pellet was suspended in a 30 mM Tris-HCl buffer solution (pH 7.4) containing 2.5 mM calcium chloride dihydrate and centrifuged at 48,000×g for 15 minutes. The supernatant was removed and the pellet was suspended in a 30 mM Tris-HCl buffer solution (pH 7.4) containing 2.5 mM calcium chloride dihydrate and after incubation at 37 °C for 30 minutes, the suspension was centrifuged at 48,000×g for 15 minutes. The resulting supernatant was removed and the pellet was suspended in a 20 mM HEPES buffer solution (pH 7.4) containing 100 mM sodium chloride and 10 mM magnesium chloride to give a membrane fraction. The membrane fraction (the protein content in the final reaction mixture: 20 µg), GDP (final concentration: 300 µM), adenosine deaminase (final concentration: 1 U/mL), R(-)-α-methyl histamine (final concentration: 300 nM) and a test drug were mixed and subjected to reaction at 30 °C for 20 minutes. After the end of the reaction, [³⁵S]GTP-γ-S (final concentration: 0.3 nM) was added and the reaction was continued for an additional 90 minutes. After the end of the reaction, the reaction mixture was suction filtered through a 96-well GF/C filter plate, which was then washed three times with a 20 mM HEPES buffer solution (pH 7.4) containing 100 mM sodium chloride and 10 mM magnesium chloride. After the washing, the filters were dried and a scintillator was added to measure the residual radioactivity on the filter with TopCount (PerkinElmer).

The residual radioactivity in the absence of R(-)-α-methyl histamine was taken as indicative of nonspecific binding and the difference from the residual radioactivity in the presence of R(-)-α-methyl histamine was taken as indicative of specific binding. Each of the test drugs was dissolved and diluted in DMSO at varying concentrations to plot a dose-response curve from the corresponding residual radioactivities; the concentration of test drug that inhibited the specific binding by 50% (IC₅₀) was determined from this curve. As it turned out, Compound No. 8 of the present invention showed high activities, i.e., IC₅₀ of 100 nM or less.

### (Test 3: Rat in vivo pharmacokinetic study)

SD rats were given a single oral administration of Compound No. 8, 19 or 22 at a dose of 3 mg/kg and an hour after the administration, the compound's distribution among the plasma, brain, and cerebrospinal fluid was checked. Quantification was effected by a high-performance liquid chromatography/tandem mass spectrometer API 4000 (LC-MS/MS; AB Sciex). As it turned out, Compound No. 8 had a brain/plasma movement ratio of 3.0, a cerebrospinal fluid/plasma movement ratio of 0.2, an intracerebral concentration of 45.7 ng/g and a cerebrospinal fluid concentration of 2.9 ng/mL. Compound No. 19 had a brain/plasma movement ratio of 4.4, a cerebrospinal fluid/plasma movement ratio of 0.7, an intracerebral concentration of 820 ng/g and a cerebrospinal fluid concentration of 134 ng/mL. Compound No. 22 had a brain/plasma movement ratio of 1.5, a cerebrospinal fluid/plasma movement ratio of 0.5, an intracerebral concentration of 209 ng/g and a cerebrospinal fluid concentration of 67.6 ng/mL.

### (Test 4: P-glycoprotein substrate recognition test)

LLC-GA5-COL300 cells (Human MDR1 expressing system derived from pig kidney derived, cultured renal epithelial cell line LLC-PK1) were cultured on a transwell. Immediately before the test, the culture medium was replaced by Hank's balanced salt solution (HBSS) and the test was then conducted. A solution of an assay compound adjusted to a final concentration of 10 µM was added to the donor side of LLC-GA5-COL300 cells and after the passage of a predetermined period of time, a specified quantity of cells was sampled from the acceptor side. The concentration of the assay compound in the sample was measured by LC-MS/MS. From the amounts of the compound that accumulated and passed into the acceptor side, membrane permeation coefficients (×10⁻⁶ cm/sec) were calculated for apical→basal and basal→apical directions and their relative ratio (efflux ratio) was determined to evaluate the P-glycoprotein substrate recognition. The efflux ratio values of Example Compounds are listed in Table 4 below.

**[Table 4]**

| Compound No. | Efflux Ratio |
|---|---|
| 8 | 1.0 |
| 19 | 0.7 |
| 22 | 1.1 |

### INDUSTRIAL APPLICABILITY

According to the present invention, there can be provided pharmaceutical products that have a potent action for inhibiting the binding to the histamine H3 receptor and which are useful in the prevention or treatment of disorders due to the histamine H3 receptor, for example, such diseases as dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasomnia, sleep related movement disorder, insomnia, depression, or allergic rhinitis and this is expected to make a great contribution to the development of the pharmaceutical industry.

## Claims

1. A compound represented by formula (I): 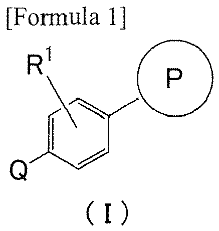 [wherein
ring P refers to a group represented by the following formula (II) or (III): 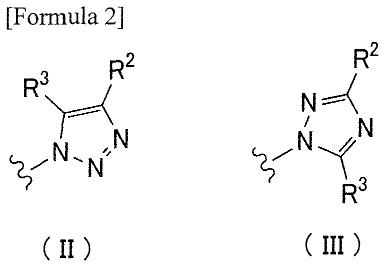 Q refers to a group represented by the following formula (A) or (B): 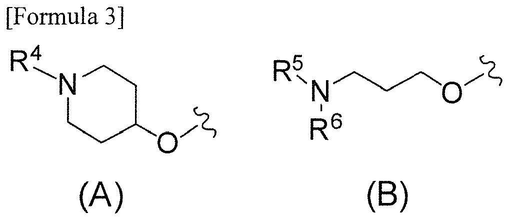 R¹ is a hydrogen atom, a halogen atom, or C₁-C₆ alkyl;
R² is a hydrogen atom, hydroxy, cyano, carboxy, C₁-C₆ alkyl, C₂-C₇ alkanoyl, C₁-C₆ alkylsulfonyl (the C₁-C₆ alkyl, C₂-C₇ alkanoyl or C₁-C₆ alkylsulfonyl may be substituted by one to three groups selected from the group consisting of a halogen atom, hydroxy and C₁-C₆ alkoxy), C₁-C₆alkoxy, C₃-C₇ cycloalkyl, C₂-C₇ alkoxycarbonyl (the C₁-C₆ alkoxy, C₃-C₇ cycloalkyl, or C₂-C₇ alkoxycarbonyl may be substituted by one to three groups selected from the group consisting of a halogen atom, hydroxy, C₁-C₆ alkyl and C₁-C₆ alkoxy), -(CH₂)ₙ-C(=O)NR^{1A}R^{1B}, or -S(=O)₂NR^{2A}R^{2B};
R^{1A} and R^{1B}, which may be the same or different, are each a hydrogen atom, C₁-C₆ alkyl or C₃-C₇ cycloalkyl, or
R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl);
n is 0 or 1;
R^{2A} and R^{2B}, which may be the same or different, are each a hydrogen atom, C₁-C₆ alkyl or C₃-C₇ cycloalkyl, or
R^{2A} and R^{2B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl);
R³ is a hydrogen atom or C₁-C₆ alkyl;
R⁴ is C₁-C₆ alkyl (the C₁-C₆ alkyl may be substituted by one or two C₃-C₇ cycloalkyls) or C₃-C₇ cycloalkyl (the C₃-C₇ cycloalkyl may be substituted by one or two C₁-C₆ alkyls); and
R⁵ and R⁶, which may be the same or different, are each C₁-C₆ alkyl or C₃-C₇ cycloalkyl, or R⁵ and R⁶ may be bonded together with the adjacent nitrogen atom to form a 3- to 7-membered saturated heterocyclic ring (the saturated heterocyclic ring being optionally substituted by one or two C₁-C₆ alkyls)] (provided that N,N-dimethyl-3-[4-(1H-1,2,4-triazol-1-yl)phenoxy]propan-1-amine is excluded) or a pharmaceutically acceptable salt thereof.

2. A compound according to claim 1, wherein the ring P is represented by formula (II): 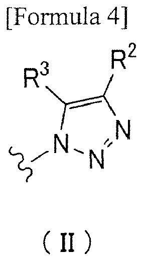 (wherein R² and R³ are as defined in claim 1) or a pharmaceutically acceptable salt thereof.

3. A compound according to claim 1 or 2, wherein Q is represented by formula (A): 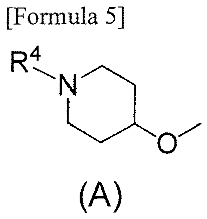 (wherein R⁴ is as defined in claim 1) or a pharmaceutically acceptable salt thereof:

4. A compound according to any one of claims 1 to 3, wherein R¹ is a hydrogen atom, or a pharmaceutically acceptable salt thereof.

5. A compound according to any one of claims 1 to 4, wherein
R² is C₁-C₆ alkyl (the C₁-C₆ alkyl may be substituted by one to three groups selected from the group consisting of hydroxy and C₁-C₆ alkoxy), C₁-C₆ alkoxy, C₃-C₇ cycloalkyl (the C₁-C₆ alkoxy or C₃-C₇ cycloalkyl may be substituted by one to three groups selected from the group consisting of hydroxy, C₁-C₆ alkyl and C₁-C₆ alkoxy) or -C(=O)NR^{1A}R^{1B}; and
R^{1A} and R^{1B}, which may be the same or different, are each a hydrogen atom, C₁-C₆ alkyl or C₃-C₇ cycloalkyl, or
R^{1A} and R^{1B} may be bonded together with the adjacent nitrogen atom to form a 3- to 7-membered saturated heterocyclic ring (the saturated heterocyclic ring being optionally substituted by one or two groups selected from the group consisting of a halogen atom and C₁-C₆ alkyl), or a pharmaceutically acceptable salt thereof.

6. A compound according to any one of claims 1 to 5, wherein R³ is a hydrogen atom, or a pharmaceutically acceptable salt thereof.

7. A compound according to any one of claims 1 to 6, wherein R⁴ is C₃-C₇ cycloalkyl, or a pharmaceutically acceptable salt thereof.

8. A pharmaceutical agent comprising as the active ingredient a compound according to any one of claims 1 to 7 or a pharmaceutically acceptable salt thereof.

9. A pharmaceutical agent according to claim 8 which is a histamine H3 receptor antagonist or inverse agonist.

10. A pharmaceutical agent according to claim 8 or 9 which is a preventive or therapeutic agent for dementia, Alzheimer's disease, attention-deficit hyperactivity disorder, schizophrenia, epilepsy, central convulsion, obesity, diabetes mellitus, hyperlipidemia, narcolepsy, idiopathic hypersomnia, behaviorally induced insufficient sleep syndrome, sleep apnea syndrome, circadian rhythm disorder, parasomnia, sleep related movement disorder, insomnia, depression, or allergic rhinitis.
